# EUROPEAN PATENT SPECIFICATION

(11) **EP 1 086 372 B1**
(45) Date of publication and mention of the grant of the patent: **31.05.2006**
(21) Application number: 99914312.6
(22) Date of filing: 29.03.1999
(51) Int. Cl.: G01N 33/558

(54) **COLLECTION DEVICE FOR SINGLE STEP ASSAY OF ORAL FLUIDS**
SAMMELAPPARAT FÜR EINSCHRITTIGEN TEST VON MUNDFLÜSSIGKEITEN
DISPOSITIF DE RECUEIL POUR DOSAGE DE FLUIDES EN UNE SEULE OPERATION

(30) Priority: 30.03.1998 US 79958 P
(43) Date of publication of application: 28.03.2001
(73) Proprietor: OraSure Technologies, Inc., Bethlehem, PA 18015 (US)
(72) Inventor: MINK, Ronald, William, West Linn, OR 97068 (US); GOLDSTEIN, Andrew, Sherman, Portland, OR 97225 (US); BOHANNON, Robert, C., Tigard, OR 97223 (US)
(74) Representative: Vossius & Partner
(86) International application number: PCT/US1999/007124
(87) International publication number: WO 1999/050656

(56) References cited:
- US-A- 4 635 488
- US-A- 4 956 302
- US-A- 5 334 502
- US-A- 5 726 010

## Description

This invention relates to the assay of oral fluids typically lateral strip chromatography. A single unit, continuous in-line, one step rapid assay format suitable for oral specimen collection and testing is disclosed. More particularly, a hydrophilic capillary matrix is disclosed as a transport for oral fluids to a lateral chromatographic strip. This enables rapid assay of oral fluids while a disposable testing device is held in a patient's mouth.

Numerous analytical methods have been developed for determining the presence or absence and/or quantifying the amount of various analytes in tissues and fluids of organisms. Currently most diagnostic testing is done with either blood, urine, fecal material, or tissue biopsy. Testing based on these materials, however, entails substantial invasion of privacy and poses a significant safety hazard (particularly with testing of blood). In contrast, the collection of oral fluid including saliva and/or mucosal transudate for testing entails relatively little invasion of privacy, is relatively safe, and can be accomplished rapidly with relative ease.

The idea for using oral fluid in a detection method has been discussed in scientific and clinical research for some time. A multitude of researchers have investigated using oral fluid as a possible clinical specimen for diagnosis of specific disease states or altered metabolic activity (*see, e.g., Annl. New York Acad. Sci., Vol. 694: Saliva as a Diagnostic Fluid,* Malamud and Tabak, eds. , N.Y. Acad. Sci. Pub. (1993)). There is a preponderance of evidence that suggests that oral fluids might be extremely useful samples for the detection of certain analytes. The basic technological premise is that analytes present in blood will pass through the oral mucosa and/or salivary glands into the oral cavity where they can be detected. Further it is assumed that the concentration of analyte in oral fluid will be indicative of the blood concentration. There is thus considerable interest in the development of devices for the collection, transport, and sample handling of oral fluids and in the development of oral fluid-based assays; in particular assays for various antibodies and metabolites.

Typically in tests performed with samples such as blood, urine, or fecal material, there is an ample supply of test material and high volumes of analytes are available for assay. In addition, since assays of such materials are performed outside the body, there is no issue of contamination of the body with assay reagents.

This is illustrated, for example, in the assay device described by de Zoeten et al US Patent 5,611,995 issued March 18, 1997. In this device an absorbing body is supplied with a handle and held in the stream of urine being expelled from the body. When the absorbing body is saturated, it is then inserted into a holding device having a test strip. The saturated pad comes into contact with a test strip, is compressed, and deposits urine to be tested on the test strip. A gap at the side of the hold device holding the test strip assures evaporation of excess fluid to prevent backflow along the test strip.

Previously described assays of biological samples, in particular assays for analytes in oral fluid, have typically required at least two different actions. First the sample, *e.g.,* blood, or urine is collected. Then the collected sample is either stored, *e.g*., for later assay in a laboratory, or is assayed in or by an assay device which is typically a device other than the collection device. Such assays, requiring multiple components, are often expensive to manufacture and cumbersome for home use.

Moreover, particularly with respect to assaying oral fluid samples, oral fluid is often in short supply, particularly under circumstances where the test subject is stressed (*e.g*., when testing for drugs of abuse or life-threatening illnesses, which may make it difficult to use such multi-component assays. In addition, attempts to stimulate oral fluid production (*e.g*., by the use of citric acid or other salivation agents) result in increased saliva production which may actually dilute the analyte concentration.

When typical absorbant pads are used to recover oral fluid, the pads must typically be compressed to release the trapped oral fluid. The manipulations associated with the compression step can result in sample contamination. In addition, such "traditional" pads have a significant void volume requiring that the sample often be collected in a volume significant greater than is actually required for analyte assay itself

The present invention is specified in the claims.

This invention provides improved devices and methods for one-step collection of oral fluid an detection and/or quantification of analytes in the oral fluid. The devices and methods require extremely low volumes of oral fluid, and require no subsequent sample manipulation after collection. Adequate sample collection is immediately verified and the risk of sample contamination is minimized. The assays are direct, rapid, and require no complicated steps. The devices and methods are therefore ideally suited for use in homes, in work or office settings, and generally do not require the presence of trained medical personnel.

Unlike prior art oral fluid collection devices that typically utilize an absorbant pad made of paper, cellulose, cotton or sponge and which require compression of the collection pad to release the oral fluid sample, the devices of this invention utilize a relatively rigid capillary matrix also referred to as a capillary matrix. The capillary matrix, when inserted into the oral cavity of a mammal (*e.g*., a human) rapidly wicks up oral fluid (*e.g*., via capillary action) and delivers it to the receiving area of a lateral flow chromatography strip. The oral fluid is rapidly released from the capillary matrix to the lateral flow chromatography strip without any manipulation (*e.g*., compression) of the matrix.

In one embodiment this invention provides an apparatus for lateral flow chromatography of an oral fluid. The apparatus comprises a capillary matrix having exposed a surface for insertion into an oral cavity; and a lateral flow chromatography strip where the lateral flow chromatography strip is attached to the capillary matrix such that when the capillary matrix is contacted to an oral mucosa in an oral cavity, the capillary matrix wicks up oral fluid and delivers the oral fluid to a receiving area of a lateral flow chromatography strip. In another embodiment, the apparatus comprises a capillary matrix having exposed a surface for receiving oral fluid; and a lateral flow chromatography strip where the lateral flow chromatography strip is in communication with the capillary matrix such that when the capillary matrix receives oral fluid, the capillary matrix wicks up the oral fluid and delivers the oral fluid to a receiving area of said lateral flow chromatography strip

In a preferred embodiment, the capillary matrix is composed of a material different from the material comprising the lateral flow chromatography strip or the receiving area or sample pad of such a strip. The capillary matrix is composed of a material such that saturation of the capillary matrix with an oral fluid does not substantially alter the morphology of the capillary matrix. Thus, neither the average pore size nor the void volume of the capillary matrix is substantially altered. In addition, the volume of the capillary matrix is substantially constant. Saturation of the capillary matrix typically effects a volume change less than less than 30%, preferably less than 25%, more preferably less than 20% and most preferably less than about 15%, 10%, 5% or less than about even 1%. The capillary matrix preferably has an average pore size ranging from about 40 µm to about 250 µm, more preferably from about 60 µm to about 200 µm, and most preferably from about 80 µm to about 120 µm, and a void volume of less than about 60 µL/cm³. Particularly preferred porous matrix materials have pore sizes that range from about 45µm to about 90 µm from about 90 µm to about 130 µm, or from about 80µm to 120 µm. Prefered capillary matrix materials are plastics (e.g., porous matrices of a high density polyethylene (HDPE), an ultra-high molecular weight polyethylene (UHMW), a polypropylene (PP), a polyvinylidene fluoride (PVDF), a polytetrafluoroethylene (PT7E), a nylon 6 (N6), or a polyethersulfone (PES)). The plastics may be hydrophilic or treated (e.g., with a surfactant such as sodium N-methyl cocoyl taurate) to be hydrophilic.

In a preferred embodiment, the capillary matrix, when contacted to an oral mucosa takes up oral fluid from said oral cavity and readily releases the oral fluid to said receiving area of said lateral flow chromatography strip in under about 1 minute without compression, altered air or fluid pressure, or other manipulation of the matrix material. The deliver comprises about 100 :L to about 200 :L of oral fluid to delivered to said lateral flow chromatography strip in under about 1 minute. The capillary matrix, when contacted to an oral mucosa takes up oral fluid from the oral cavity and releases said oral fluid to said receiving area of said lateral flow chromatography strip preferably in under about 30 seconds. Under these conditions, the capillary matrix, is preferably saturated with oral fluid in under about 1 minute, and saturation typically utilizes less than about 500 µL of oral fluid. Generally speaking, the capillary matrix will release sufficient oral fluid to saturate the receiving area of the chromatographic strip.

The apparatus can optionally further include a blocking strip placed between the capillary matrix and the lateral flow chromatographic strip. The blocking strip can contain a blocking reagent (*e.g.,* BSA, deoxycholate, sodium -n-lauroylsarcosine, *etc.*) and/or one or more buffers. The blocking strip can also prevent backflow of reagents from the lateral flow chromatography strip to the capillary matrix.

The apparatus can optionally further include a conjugate strip that contains one or more chromatography reagents (*e.g.,* labeled microparticles). In addition, a single strip can double as a blocking strip and a conjugate strip.

The apparatus can further comprise a a housing having a cavity, wherein said lateral flow chromatography strip extends into the cavity along the housing to an inspection site on the housing; and at least one inspection site from an exterior of the housing to the lateral chromatographic strip to enable visual inspection of reagents at selected sites on the lateral chromatographic strip. The housing can act as a handle for inserting the capillary matrix into the oral cavity.

In one particularly preferred embodiment, the assay device comprises a single unit, continuous in-line, one step rapid assay format suitable for oral specimen collection and testing is disclosed. More particularly, a hydrophilic capillary matrix is provided as a transport for oral fluids to a lateral chromatographic strip. The lateral chromatographic strip is placed within a cavity defined in a housing and is disposed along the housing to an inspection site. A hydrophilic capillary matrix protrudes from the housing to an oral collection site exterior of the housing at one end and communicates to the lateral chromatographic strip at the other end. This hydrophilic capillary matrix defines a matrix of passageways defined between non-absorbent materials, such a either plastic spheres or foams. The exterior surfaces of the matrix are hydrophilic by either being naturally hydrophilic or treated to be hydrophilic. Interstitial matrix dimension is such that forces of capillary action cause the immediate drawing of materials into the hydrophilic capillary matrix. The hydrophilic capillary matrix readily releases oral fluid to the lateral chromatographic strip. Prevention of reverse flow to the oral cavity from the lateral chromatographic strip naturally occurs due to the circuitous flow path of the porous wick material. By observing the lateral chromatographic strip while the entire test device is in the mouth immediate test results are obtained.

In another embodiment, this invention provides a method of detection or quantifying one or more analytes in an oral fluid. The method involves the steps of: i) inserting into the oral cavity of a mammal any of the oral fluid assay apparatuses described herein such that the capillary matrix is contacted with an oral mucosal surface whereby the capillary matrix wicks up oral fluid and delivers the oral fluid to a receiving area of a lateral flow chromatography strip; and ii) reading a signal on the lateral flow chromatography strip that indicates the presence absence or quantity of one or more analytes.

This invention also provides kits for the detection of an analyte in an oral fluid. The kits include an apparatus for collection and lateral flow chromatography of an oral fluid as described herein and instructional materials describing the use of the apparatus.

### Definitions.

As used herein, the term "analyte" is used to refer to a moiety that is to be detected in a particular assay. Analytes can be atoms (elements), molecules, or groups of molecules. Analytes commonly detected in the assays of this invention include, but are not limited to antibodies, antigens, growth factors, enzymes, therapeutic drugs, drugs of abuse, and the like. Particularly preferred analytes include antibodies and antigens relevant to infectious and non-infectious disease.

As used herein, an "antibody" refers to a protein consisting of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively.

The basic immunoglobulin (antibody) structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies may exist as intact immunoglobulins or as a number of well characterized fragments produced by digestion with various peptidases. Thus, for example, pepsin digests an antibody below the disulfide linkages in the hinge region to produce F(ab)'₂, a dimer of Fab which itself is a light chain joined to V_{H}-C_{H}1 by a disulfide bond. The F(ab)'₂ may be reduced under mild conditions to break the disulfide linkage in the hinge region thereby converting the F(ab)'₂ dimer into an Fab' monomer. The Fab' monomer is essentially an Fab with part of the hinge region (see, *Fundamental Immunology,* W.E. Paul, ed., Raven Press, N.Y. (1993) for a more detailed description of other antibody fragments). While various antibody fragments are defined in terms of the digestion of an intact antibody, one of skill will appreciate that such Fab' fragments may be synthesized de novo either chemically or by utilizing recombinant DNA methodology. Thus, the term antibody, as used herein also includes antibody fragments either produced by the modification of whole antibodies or synthesized *de novo* using recombinant DNA methodologies.

The term "oral fluid", as used herein, refers to one or more fluids found in the oral cavity individually or in combination. These include, but are not limited to saliva and mucosal transudate. It is recognized that oral fluid (e.g., saliva) can comprise a combination of fluids from a number of sources (e.g., parotid, submandibular, sublingual, accessory glands, gingival mucosa and buccal mucosa) and the term oral fluid includes the fluids from each of these sources individually, or in combination. The term saliva refers to a combination of oral fluids such as is typically found in the mouth, in particular after chewing. The term "mucosal transudate", as used herein, refers to fluid produced by the passive diffusion of serum components from oral mucosal interstitia into the oral cavity. Mucosal transudate often forms one component of saliva.

The terms "capillary matrix" or "porous matrix" are used herein to refer to a a highly porous material characterized by a pore size sufficiently small that the material rapidly takes up aqueous solution (e.g. of oral fluid) predominantly by capillary action or "wicking".

The term "wick up" is used to refer to the uptake of a fluid predominantly by adsorption and capillary action.

A "lateral flow chromatography strip" refers to a test strip utilized for lateral flow chromatography. Lateral flow (chromatography) assays typically involve the application of a liquid test sample suspected of containing an analyte to be detected to an application zone of an lateral flow (immunochromatographic) test strip . The strip is comprised of a matrix material (*e.g*., paper, nitrocellulose, *etc*)*., see, e.g.,* U.S. Patent. No. 5569608) through which the test fluid and analyte suspended or dissolved therein can flow by capillary action from the application zone to a detection zone where a visible signal, or absence of such, reveals the presence or absence of the analyte. Where the detection of the analyte utilizes an antibody or antibody fragment, the assay may be referred to as a lateral flow immunochromatography assay and the strip a lateral flow immunochromatography strip.

A "receiving area or sample pad of said lateral flow chromatography strip" refers to the area of the lateral flow chromatography strip to which a sample is first applied.

A "signal on said lateral flow chromatography strip" refers to an indication, typically in a particular predefined region of the chromatography strip that indicates the presence or absence or quantity of analyte or the sufficiency of sample within the chromatography strip. The signal can be colorimetric, fluorescent, electroluminescent, radioactive, etc.

As used herein, "housing" refers to any member which encases or supports but does not react with the lateral flow chromatographic strip.

As used herein, "cavity" refers to any receiving volume on or within the housing for holding the lateral chromatographic strip.

The "lateral flow chromatographic strip" is any absorbing member capable of transporting analyte and reagents to the visual inspection site. The strip can be nitro cellulose, cellulose acetate, paper, nylon, cellulose or any other suitable bibulous material.

As used herein, an "immunoassay" is an assay that utilizes an antibody or antigen to specifically bind to the analyte. The immunoassay is characterized by the use of specific binding to a particular antibody as opposed to other physical or chemical properties to isolate, target, and quantify the analyte.

The phrase "specifically binds to an analyte" or "specifically immunoreactive with", when referring to an antibody refers to a binding reaction which is determinative of the presence of the analyte in the presence of a heterogeneous population of molecules such as proteins and other biologics (*i.e*., such as may be found in oral fluid). Thus, under designated immunoassay conditions, the specified antibodies bind to a particular analyte and do not bind in a significant amount to other analytes present in the sample. A variety of immunoassay formats may be used to select antibodies specifically immunoreactive with a particular analyte. For example, solid-phase ELISA immunoassays are routinely used to select monoclonal antibodies specifically immunoreactive with a protein. See Harlow and Lane (1988) *Antibodies, A Laboratory Manual,* Cold Spring Harbor Publications, New York, for a description of immunoassay formats and conditions that can be used to determine specific immunoreactivity.

A "label" is a composition detectable by spectroscopic, photochemical, biochemical, immunochemical, electrical, optical or chemical means. Useful labels in the present invention include magnetic beads (*e.g*. DynabeadsTM), fluorescent dyes (*e.g*., fluorescein isothiocyanate, texas red, rhodamine, green fluorescent protein, and the like), radiolabels (*e.g*., ³H, ¹²⁵I, ³⁵S, ¹⁴C, or ³²P), enzymes (*e.g*., horse radish peroxidase, alkaline phosphatase and others commonly used in an ELISA), and colorimetric labels such as colloidal gold or colored glass or plastic (*e.g*. polystyrene, polypropylene, latex, *etc.*) beads. Patents teaching the use of such labels include U.S. Patent Nos. 3,817,837; 3,850,752; 3,939,350; 3,996,345; 4,277,437; 4,275,149; and 4,366,241. Means of detecting such labels are well known to those of skill in the art. Thus, for example, radiolabels may be detected using photographic film or scintillation counters, fluorescent markers may be detected using a photodetector to detect emitted illumination. Enzymatic labels are typically detected by providing the enzyme with a substrate and detecting the reaction product produced by the action of the enzyme on the substrate, and colorimetric labels are detected by simply visualizing the colored label.
Fig. 1 is a schematic embodiment of the oral collection device of this disclosure illustrating generally from left to right a wick for the transport of oral fluids, a blocker pad, the gold conjugate pad, the lateral chromatographic strip with observation window, and finally an end absorption pad all contained within a simple housing;
Fig. 2 is a schematic embodiment of an alternate oral collection device similar to Fig. 1 omitting the blocking pad and having the gold conjugate applied to the end of the wick material; and,
Fig. 3 is a three dimensional rendering of the test device with wick cover being withdrawn for use in the oral cavity of a human.

This invention provides a device for the rapid, one-step, collection and detection of analytes in oral fluid. In a preferred embodiment, the device is inserted into the oral cavity (*e.g*., preferably juxtaposed to the oral mucosa) where it absorbs oral fluid. After a period of time, the device is removed from the oral cavity and one or more indicators contained in the device are read out (*e.g.,* by visual inspection or by detection in a "reader") to provide an indication of the presence or absence, and/or quantity of one or more analytes of interest. The device thereby provides a rapid, one-step, non-invasive assay for the detection of one or more analytes of interest.

The assay devices and methods of this invention can be used for the detection (positive or negative, and/or quantification) of virtually any analyte in oral fluid. Moreover, the devices and methods can be used to detect one or more analytes simultaneously. Such analytes may include, but are not limited to, antibodies to HIV, antibodies to HTLV, antibodies to *Helicobacter pylori,* antibodies to hepatitis, antibodies to measles, antibodies to mumps, antibodies to rubella, cotinine, cocaine, benzoylecgonine, benzodizazpine, tetrahydrocannabinol, nicotine, ethanol theophylline, phenytoin, acetaminophen, lithium, diazepam, nortryptyline, secobarbital, phenobarbitol, theophylline, testosterone, estradiol, 17-hydroxyprogesterone, progesterone, thyroxine, thyroid stimulating hormone, follicle stimulating hormone, luteinizing hormone, transforming growth factor alpha, epidermal growth factor, insulin-like growth factor I and II, growth hormone release inhibiting factor, IGA and sex hormone binding globulin; and other analytes including glucose, cholesterol, caffeine, cholesterol, corticosteroid binding globulin, PSA, or DHEA binding glycoprotein with the methods being particularly well suited to the detection of HIV antibodies.

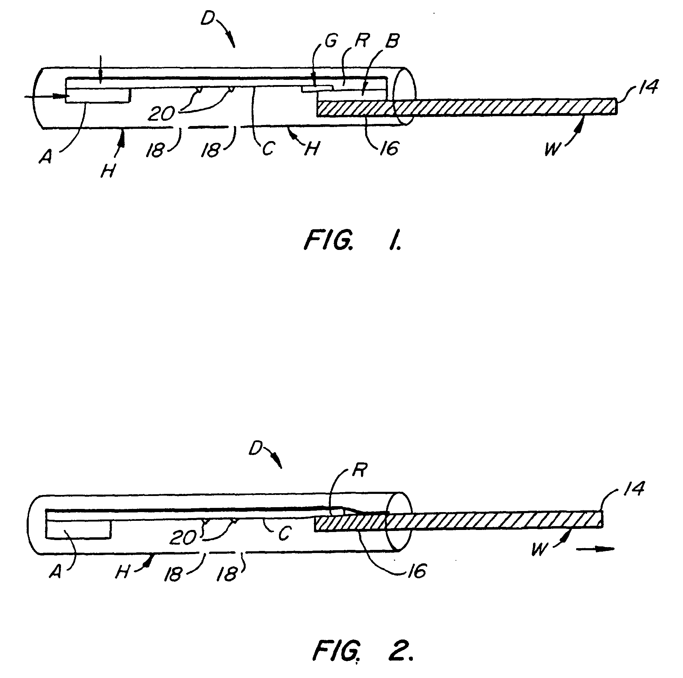

The assay device of this invention relies on the unique cooperation between a capillary matrix (W in Figure 1) and a lateral flow chromatographic strip (C in Figure 1). The device is constructed such that the capillary matrix can be inserted into the oral cavity, and, in a preferred embodiment, juxtaposed to the oral mucosa. The capillary matrix acts as a receiving body or pad that rapidly absorbs (wicks up) oral fluid, *e.g.,* via capillary action, and delivers that oral fluid to a lateral flow immunochromatography strip (*e.g*., C in Figure 1). The immunochromatography strip then provides an indication of the presence, absence or quantity of one or more analytes in the oral fluid.

The device can be conveniently assembled such that the capillary matrix comprises a receiving pad for insertion into the oral cavity, while the lateral flow immunochromatography strip comprises the handle of the device. One or more zones on the handle can comprise indicators for readout (*e.g.*, of a colorimetric signal) of the assay results. Of course, other formats of the device are suitable and will be readily recognized by those of skill in the art.

### Capillary Matrix.

The capillary matrix (porous matrix) material is preferably selected to provide a number of unique properties to the assay device. Such properties include, but are not limited to, a relatively low void volume, a pore size sufficient to provide rapid and effective delivery of the oral fluid to the test strip, low or non-reactivity with the oral fluid or analytes, easy release of the oral fluid to the immunochromatography test strip, and a non-deformable (when wetted) collection pad.

Because oral fluid may be in short supply (patients often suffer a "dry mouth" during testing) it is desirable to maximize the amount of oral fluid that is transported from the oral cavity (*e.g*., the oral mucosa) to the lateral flow chromatography strip. This is accomplished by the use of a capillary matrix having a minimum void volume. The capillary matrix should have a void volume less than about 65%/cm³, preferably less than about 57%/cm³, more preferably less than about 48%/cm³, and most preferably less than about 40%/cm³, 35% cm³, or even 25% cm³. Capillary matrices having such low void volumes typically deliver a significant amount of the absorbed oral fluid to the lateral flow chromatography strip.

The matrix itself must be of relatively small dimension. Specifically, the interstices is preferably of a dimension where capillary forces cause the fluid to be drawn into the capillary matrix. Thus, the capillary matrix is also selected to have an average pore size small enough to provide rapid uptake of the oral fluid with which it is contacted (*e.g*., via capillary action). The small pore size also functions to exclude particulate material present in the fluid sample. The pore size, however, is also selected to be large enough that the viscous oral fluid does not clog the capillary matrix and instead rapidly transports through the matrix to the lateral flow chromatography pad. Preferred materials have an average pore size that ranges from about 40 µm to about 250 µm, more preferably from about 60 µm to about 200 µm, and most preferably from about 80 µm to about 120 µm.

In addition to having a pore size (channel size) that results in rapid uptake of the oral fluid, the surfaces of the capillary matrix should be chemically compatible with rapid uptake of the oral fluid. Thus, preferred capillary matrix materials are themselves hydrophilic or treated to be hydrophilic (*e.g*. by addition of a surfactant also referred to as a detergent or wetting agent). That is to say, water must flow on and be attracted to the surfaces of these materials.

While a number of suitable materials are naturally hydrophillic (e.g. clean scintered glass or fused glass beads), other suiutable materials (*e.g.,* plastics) are typically hydrophobic (*e.g.,* do not easily wet). However, such hydrophobic materials can be routinely treated with a wetting agent (*i.e.,* surfactant/detergent) and thereby rendered hydrophilic (wettable). However, since the capillary matrix is used in the oral cavity, it is required that the treating detergent be known not to be harmful to the subject mammal (e.g., human body) and preferably be approved for such use by the relevant regulatory authority (*e.g.*, Food and Drug Administration). In one preferred embodiment, a porous plastic material (*e.g.*, polyethylene or polypropylene foam) can be rendered hydrophilic by taking the untreated matrix material and placing it in a dilute aqueous solution of an approved detergent such as sodium N-methyl cocoyl taurate. Thereafter, the treated material is dried, leaving the surfaces of the matrix apparently thinly coated with the detergent.

While N-methyl cocoyl taurate is preferred, it will be appreciated that other detergents can as well be used. It is only required that the detergent by safe for mammalian oral exposure, not interfere with the test on lateral chromatographic strip C, and produce the required hydrophilic properties on the exterior surfaces of the matrix.

In addition to rapidly taking up and transporting the oral fluid to the lateral flow chromatography strip, the capillary matrix material is selected that preferably readily releases the fluid to the chromatography strip. This should be accomplished rapidly without compression of the matrix material itself. Thus, in a preferred embodiment, the capillary matrix delivers and releases oral fluid to the lateral flow chromatography strip with no manipulation (*e.g*. no squeezing or compression of the capillary matrix).

From the foregoing, it should be clear that preferred capillary matrix materials have an interstitial spacing that facilitates uptake of oral fluid through capillary attraction in combination with adsorption on the material. This causes the oral fluid gathered from the mouth to be transported to the lateral chromatographic strip in preference to remaining in the mouth. At the same time, when the oral fluid arrives at the lateral chromatographic strip, it is absorbed to the strip in preference to remaining in the capillary matrix.

In a preferred embodiment, the hydrophilic capillary matrix is an essentially non absorbing matrix which adsorbs liquid via capillary action. In such adsorbtion, the volume of the material is not appreciably effected. In addition, the capillary matrix material is relatively rigid such that its morphology remains essentially unchanged during the assay (*e.g*. when saturated with oral fluid). Thus, saturation of the matrix with an oral fluid does not substantially alter the average pore size or void volume of the porous matrix. In addition, saturation of the capillary matrix with an oral fluid results in a volume change of less than 30%, preferably less than 25%, more preferably less than 20% and most preferably less than about 15%, 10%, 5% or less than about even 1%.

In a particularly preferred embodiment, the capillary matrix can act as a barrier to back flow of reagents from the lateral flow chromatography strip back into the capillary matrix. This can be accomplished, for example, where the chromatography strip has a larger volume for fluid storage than the capillary matrix. In addition or alternatively, where the lateral flow chromatography strip is more hydrophilic than the capillary matrix the capillary matrix can also act as a barrier to backflow.

The capillary matrix materials are selected such that they are not chemically reactive with either the oral fluid or the analytes contained therein. Matrix materials compatible with oral fluid are well known to those of skill in the art are include, but are not limited to glass, resins, and various plastics.

In one preferred embodiment, the properties described above are achieved by the use of porous plastic materials for the capillary matrix. Suitable porous plastic materials include, but are not limited to, porous matrices of high density polyethylene (HDPE) ultra-high molecular weight polyethylene (UHMW), polypropylene (PP), polyvinylidene fluoride (PVDF), polytetrafluoroethylene (PTFE), nylon 6 (N6) and polyethersulfone (PES). In a preferred embodiment, the porous matrix materials are either themselves hydrophilic (so as to readily uptake the oral fluid) or are treated (*e.g.,* with a surfactant/detergent) so as to be hydrophilic.

Such porous plastics are commercially available (*see, e.g.,* Porex Technologies, Fairburn, GA). Particularly preferred porous plastics are detergent (surfactant) treated polyethylene and/or polypropylene. The treatment typically involves soaking the capillary matrix in a surfactant/detergent and then allowing it to dry naturally or force drying the material.

Particularly preferred porous matrix materials are Porex X-4588, 80-120 µm pore size at 0.024 inches of thickness made from polypropylene. Likewise, Porex X-4903 at 0.0.0625 inches, pore size 45-90 µm, and Porex X-4913 at 0.0625, pore size 90-130 µm are suitable. In one preferred embodiment, these materials are treated with sodium N-methyl cocoyl taurate. The capillary matrix materials are soaked in the detergent which is then dried onto the surface comprising the porous matrix.

It will be understood that the Porex7 materials that are utilized do not retain large volumes of the oral fluid. For example, consider the following data:
Porex Void Volume

**Table 1. Void volume of Porex® X-4903.**

| Medium Pore | | | | |
|---|---|---|---|---|
| Porex X-4903 1 cm X 1 cm X 0.1588 cm | | | | |
| # | Dry Weight (g) | Weight+H₂O (g) | Weight of H₂O (g) | % Volume/cm³ % Volume/cm³ |
| 1 | 0.0791 | 0.1595 | 0.0804 | 50.63 |
| 2 | 0.0745 | 0.1480 | 0.0735 | 46.28 |
| 3 | 0.0746 | 0.1480 | 0.0734 | 46.22 |
| 4 | 0.0767 | 0.1503 | 0.0736 | 46.35 |
| 5 | 0.0762 | 0.1503 | 0.0741 | 46.66 |
| | | Average | 0.0750 | 47.23 |

**Table 2. Void volume of Porex® X-4913.**

| Coarse Pore | | | | |
|---|---|---|---|---|
| Porex X-4913 1 cm X 1 cm X 0.1588 cm | | | | |
| # | Dry Weight (g) | Weight+H₂O (g) | Weight of H₂O (g) | % Volume/cm³ |
| 1 | 0.0782 | 0.1670 | 0.0888 | 55.92 |
| 2 | 0.0803 | 0.1723 | 0.0920 | 57.93 |
| 3 | 0.0806 | 0.1700 | 0.0894 | 56.30 |
| 4 | 0.0851 | 0.1818 | 0.0967 | 60.89 |
| 5 | 0.0747 | 0.1593 | 0.0846 | 53.27 |
| | | Average | 0.0903 | 56.86 |

Above, we assume that 1cm³ =1 ml = 1 g H₂O.

At the same time, hydrophilic capillary matrix W does not have a high relative retention of the oral fluid. For example, it readily surrenders its fluid to lateral chromatographic strip C and absorbent pad A. It will be understood that hydrophilic capillary matrix W acts more as a conduit than as an absorbent; material is readily discharged from the wick.

### Identification of suitable porous matrix materials.

It will be appreciated that the rate of oral fluid uptake, transport to the lateral flow chromatography pad and release to the pad is a function of both the composition of the capillary matrix and its shape (*e.g*. area exposed to the oral mucosa, cross-sectional area, and area contacted to the lateral flow chromatography strip. The rates are also effected by the average pore size, hydrophilicity of the capillary matrix material and the relative absorbance characteristics of the capillary matrix and the lateral flow chromatography pad.

These parameters can be optimized according to routine methods well known to those of skill in the art. In one embodiment, this is accomplished by assembling a test device having the desired capillary matrix material and lateral flow immunochromatography strip. The test device can then be contacted with a test oral fluid solution (e.g, natural oral fluid, or synthetic oral fluid, *see,* U.S. Patent 5,695,929 and copending USSN 08/608,431).

The contacting can be accomplished by actual insertion of the receiving portion (*e.g.,* receiving pad/face) of the capillary matrix into an oral cavity and contact with an oral mucosa (*e.g.,* of a human or non-human test animal). Alternatively, the contacting can be accomplished by touching the capillary matrix with the test fluid disposed on a surface or in a bowl or other receptacle, immersing part or all of the capillary matrix in the test fluid, or contacting the capillary matrix with a test body (*e.g*.; a sponge, cloth, swab, *etc*.) impregnated with the test fluid.

The test oral fluid will typically be contacted with the capillary matrix for the time period it is desired to run the assay (*e.g*. less than 5 minutes) and then the lateral flow chromatography strip can be read for the presence, absence or quantity of analyte and/or for the amount of oral fluid taken up . In this manner it will be determined if the assay gives adequate sensitivity and specificity.

In addition, the time period for uptake of oral fluid into the capillary matrix, and lateral flow chromatography strip can be determined. Similarly the total volume of sample required to properly saturate the lateral flow chromatography strip and the amount of fluid retained by the capillary matrix can also be ascertained (*e.g*., by weighing the various components before and after the assay.

In a preferred embodiment, the capillary matrix, when inserted and held in a mouth is saturated with oral fluid in under about 5 minutes, preferably in under about 3 minutes, more preferably in under about 1 minute, and most preferably in under about 30 seconds. Similarly, the capillary matrix will release sufficient fluid to the chromatography strip for an properly run assay (an assay run to the specifications of the chromatography strip) in under about 10 minutes, preferably in under about 5 minutes, more preferably in under about 3 minutes and most preferably in under about 2 minutes or even in under about 1 minute without compression of the porous matrix.

A porous matrix will be made that is saturated by less than about 500 :1, more preferably by less than about 500 µL, preferably by less than about 500 µL, more preferably by less than about 300 µL and most preferably by less than about 100 µL.

It will be recognized that where just the oral fluid uptake and release properties are to be assayed, there is no need to utilize a complete chromatographic assay. The base capillary matrix material, lateral flow chromatography strip, and if desired, blocking pad, materials can be assembled. The rate of fluid uptake and delivery to the pad can be quantified by immersing or contacting the capillary matrix pad with a sample fluid (*e.g*., natural or synthetic oral fluid) and quantifying the rate of fluid uptake and delivery to the pad and the and/or the amount retained within the capillary matrix (*e.g*. by weighing the various elements after particular preselected times of exposure to the test fluid). A combination of materials and shapes elements that provides maximal fluid delivery of an oral fluid from an oral mucosa to the lateral flow chromatography strip in the shortest amount of time is preferred.

In one particularly preferred embodiment, the lateral flow chromatography strip is a nitrocellulose strip (*e.g*., Syntron QuickScan 6, Avitar Visualine II, Avitar Technologies, Canton, Massachusetts). A typical chromatographic strip is Millipore SRHF nitrocellulose membrane (Millipore Corp., Bedford, Massachusetts) having dimensions 4 mm x 50 mm. A porous matrix material measures 7 mm x 52 mm (0.0625 inches thick) and overlaps the nitrocellulose membrane by about 64 mm². In another embodiment, the capillary matrix is paddle shaped having a total surface area of about 720 mm² and overlapping the chromatographic strip by about 8 mm². The small overlap is particularly well suited to embodiments containing a conjugate pad a as it serves to effectively channel the oral fluid through the conjugate pad.

### Lateral flow chromatography strip.

The assay device of this invention can utilize virtually any lateral flow chromatography strip for detection and/or quantification of the analyte or analytes. Lateral flow chromatography assays are well known to those of skill in the art (*see, e.g.* U.S. Patents 5,569,608, 5,120,643, 5,656,503,4,855,240, 5,591,645, British Patent GB 2204398A, and European patent EP 0323605 B1) and such assays are commercially available on a retail or OEM basis for numerous analytes.

Lateral flow immunoassays typically involves the application of a liquid test sample suspected of containing an analyte to be detected to an application zone of an lateral flow (immunochromatographic) test strip. The strip is comprised of a matrix material (*e.g.,* paper, nitrocellulose, *etc., see, e.g.,* U.S. Patent. No. 5569608) through which the test fluid and analyte suspended or dissolved therein can flow by capillarity from the application zone to a detection zone where a visible signal, or absence of such, reveals the presence or absence of the analyte.

Typically, the strip will include means for immunospecifically binding the analyte to be detected with its specific binding partner (*e.g.,* where the analyte is an antigen, the binding partner is an antibody or antibody fragment, and *vice versa*) which bears a detectable label. In one such scheme; as disclosed in U.S. Pat. No. 4,446,232; the strip contains an enzyme labeled, mobile binding partner for the analyte which is in a zone downstream from the sample application zone. If analyte is present in the test sample, it will combine with its labeled binding partner to form a complex which will flow along the strip to a detection zone which contains a substrate for the enzyme label capable of providing a signal (*e.g*., a colored response) in the presence of the enzyme label.

The strip typically contains a zone in which analyte is immobilized, so that the labeled binding partner which does not combine with analyte, due to absence of analyte in the sample, will be captured and thereby inhibited from reaching the detection zone. There have been published various modifications of this technique, many of which involve some competitive specific binding system in which the presence or absence of analyte in the test sample is determined by the detection or lack thereof of labeled binding partner in the detection zone. In U.S. Patent No: 4,868,108 there is disclosed a similar scheme with the addition of an immobilized capture reagent for the enzyme labeled binding partner in the detection zone to concentrate the enzyme label and enhance its ability to react with the enzyme substrate and thereby render the assay more sensitive.

Not all of the schemes for immunochromatography rely on an enzyme labeled binding partner/enzyme substrate as providing the signal for detection of the analyte. In U.S. Pat. No. 4,806,311 there is disclosed a multizone test device for the specific binding assay determination of an analyte and an immobilized binding partner therefore together with a detection zone for receiving labeled reagent which migrates thereto from the reagent zone. The detection zone contains an immobilized form of a binding substance for the labeled reagent The labeled reagent bears a detectable chemical group having a detectable physical property which is detectable on the basis of its own physical properties, so that it does not require a chemical reaction with another substance. Exemplary of such groups are colored species fluorescers, phosphorescent molecules, radioisotopes and electroactive moieties. U.S. Pat. No. 4,313,734 describes the use of gold sols as labels for antibodies which are detectable without a chemical change.

Many lateral flow immunochromatography systems utilize particulate (microparticle) markers (*e.g*., gelatin, dyed latex, or colloidal gold) which are labeled with a binding partner (*e.g*., antibody or antigen) that binds the analyte of interest

The microparticles or other detectable moieties attached to an analyte binding moiety (*e.g*. an antibody or antigen) are dried onto (or otherwise localized in) either a lateral flow chromatographic strip or onto a sample application pad (typically glass fiber) which in turn is affixed to one end of a strip of chromatographic medium such as nitrocellulose. Another material binding to the analyte of interest is affixed to the chromatographic medium at or near the end opposite to the end having the application pad.

The liquid sample to be analyzed is placed on the pad, causing the suspension of the microparticles into the liquid and allowing any analyte in the liquid sample to bind to the analyte-binding material attached to the microparticles. The liquid sample leaves the application pad by diffusion and capillary action and begins to migrate along the nitrocellulose strip carrying the microparticles down the strip along with the liquid. When the liquid containing the suspended microparticles arrives at the region of the chromatographic strip bearing the second binding material, the analyte (if present in the original sample) will form a molecular bridge between the analyte-binding material on the microparticles and the analyte-binding material affixed to the strip, resulting in the immobilization of the microparticles at that point on the strip where the analyte-binding material is affixed. This immobilization of the microparticles results in a visible signal (*e.g*., a colored band or dot) at this point on the strip. If the analyte is not present in the sample, the microparticles will continue past this location on the chromatographic strip and a visible signal will not be produced.

It will be appreciated that other labels (*e.g*. fluorescent labels) besides microparticles can be utilized. In addition, a single chromatography strip can contain reagents to detect or quantify a number of different analytes.

It will also be appreciated that the lateral flow strip can use an analyte detection that does not involve an antibody-antigen recognition system. Thus, for example, the strip can be impregnated in a detection zone with a chemical that reacts with the analyte itself to produce a signal.

The devices of this invention can be readily assembled with any of a variety of commercially available lateral flow chromatography assays (*e.g*. Syntron QuickScan 6, Avitar Visualine II, Avitar Technologies, Canton, Massachusetts, etc.).

### Optional blocking strip.

The assay devices of this invention can optionally include a blocking strip between the porous matrix and the lateral flow chromatography strip. The blocking strip can be impregnated with buffers to adjust the pH (*e.g.*, to pH 7.5) of the oral fluid for compatibility with the lateral flow chromatography assay. The blocking strip can also include one or more blocking reagents that reduce non-specific binding of the analyte and/or reagents of the assay device and thereby reduce the occurrence of false positives. Suitable blocking reagents include, but are not limited to bovine serum albumin (BSA), deoxycholate, and n-lauroyl sarcosine. In one particularly preferred embodiment, the blocking solution includes: 4% polyvinyl alcohol (10 kd mw (Aldrich 36,062-2), 4% sodium -n-lauroylsarcosine (Sgima L5777), 2% polyvinyl pyrrolidone (10 kd mw, Sigma P2263) in 60 X Tris EDTA (Sigma T9285) or 7.5% sodium -n-lauroylsarcosine (Sigma L5777), 2.5% tectronic 1307 (BASF), 0.00001% polyethyleneglycol compound (Sigma P2263) in 100 X Tris EDTA buffer (Sigma T9285)..

The blocking pad can be composed of a wide variety of materials as long as they do not impede flow of oral fluid from the capillary matrix to the lateral flow chromatography strip. Such materials include, but are not limited to paper, cellulose, nitrocellulose, and the like. Particularly preferred materials will be selected to reduce or eliminate backflow of reagents or oral fluid from the chromatographic test strip to the capillary matrix. In a preferred embodiment, when present, the blocking pad is a Schleicher & Schuell # 470 cellulose filter (Schleicher & Schuell, GMBH, Germany).

### Optional Conjugate Strip.

In one embodiment, the lateral flow chromatography reagents (*e.g.,* antibody labeled gold particles, *etc.*) are disposed on or in the lateral flow chromatographic strip itself. In another embodiment, however, one or more chromatography reagents can be disposed in a conjugate strip (G), *e.g*, to facilitate fabrication. The conjugate strip is positioned juxtaposed to the lateral flow chromatography strip such that the oral fluid must pass across or though the conjugate strip in order to migrate up the chromatographic strip. Alternatively, the conjugate strip can be woven into the lateral flow chromatography strip or can be placed in-line, in the same plane, as the lateral flow chromatography strip. As with the blocking strip, the conjugate strip can be fashioned out of any convenient material (*e.g*., nitrocellulose, glass fiber, polyester, *etc*.) that is compatible with the assay and that does not substantially impede flow of the oral fluid and reagents. In a preferred embodiment, the conjugate pad is a 4 mm x 4 mm glass fiber pad or a polyester pad (*e.g*., Ahlstrom Remay # 2033)

### Assembly of the assay device.

The components (*e.g*. capillary matrix, lateral flow chromatography strip, optional blocking strip) can be assembled by any of a wide variety of means well known to those of skill in the art. Thus, for example, the components can be welded together or glued together and the like.

In a preferred embodiment, the components are simply pressed together and held in place by a housing (H). Housing H can be of any desired construction. For example, in prototype construction, soda straws were utilized which supplied both adequate structure and visibility to gauge accuracy of testing. Acrylic tubing has also been used. It will be recognized that other stock tubing materials can be used or specially designed housings can be custom molded or extruded.

The components were assembled such that the lateral flow chromatography strip was disposed lengthwise within the straw and appressed at one end to and end of the capillary matrix (W). The housing H thus provided a convenient handle for insertion of the capillary matrix into the oral cavity of a mammal (*e.g*. a human).

Figures 1, 2, and 3 illustrate various embodiments of the assay device of this invention. Lateral flow chromatography or immunochromatography strip (C) is disposed lengthwise within housing (H). One end of the chromatography strip contacts directly, or by way of blocking pad (B) with a portion of capillary matrix (W). The capillary matrix (W) projects out of the housing (H) where it presents a face (3) that acts as an absorbant surface for uptake of oral fluid. The oral fluid migrates through the matrix (W) and through blocking pad (B) if present, where it is finally delivered to a receiving area (R) on the lateral flow chromatographic strip.

The oral fluid then migrates along the lateral flow chromatography strip where it interacts with various reagents (*e.g*., antibody or antigen labeled binding partners (*e.g*., antibodies or antigens)) that are deposited within the chromatography strip and/or within optional conjugate pad G which, when present, contacts the chromatography strip.

The oral fluid/reagent combination continues to migrate along the chromatography strip until it reaches one or more indicator zones (20). If an analyte is present the indicator zone immobilizes the bound labeled analyte or otherwise produces a detectable signal. One or more of the indicator zones can also indicate sample sufficiency (*e.g*., with a color change) when the necessary sample volume and/or analyte concentration is reached. Sample sufficiency indicators are well known to those of skill in the art and particular advantageous sample sufficiency indicators are described in copending application USSN 08/456,459 and in U.S. Patent 5,479,937).

The indicators zones can be read (*e.g*. via visual inspection or other means) through view ports (18). The device is optionally equipped with blocking pad (B). This pad can be impregnated with a blocking reagent (*e.g*., n lauryl sarcosine) and/or buffer(s) for adjusting pH of the sample. In addition, the blocking pad can be fashioned out of a semipermeable material that allows oral fluid flow towards the chromatography strip, but prevents backflow of oral fluid and/or reagents into the capillary matrix. The far end (away from the capillary matrix) of the chromatography strip can bear affixed thereto an absorbant pad which acts as a reservoir to receive the oral fluid and thereby prevent backflow into the capillary matrix.

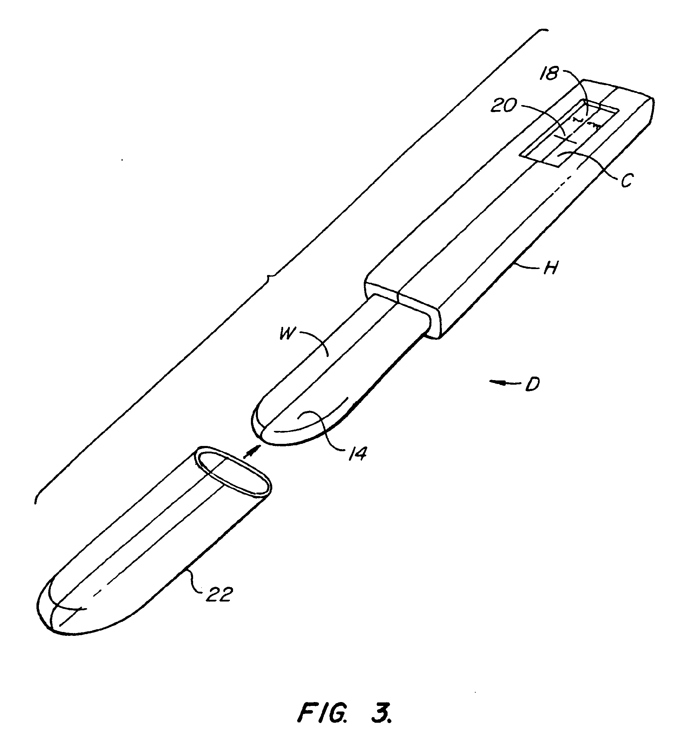

It will be appreciated that the devices can be assembled in a wide variety of forms. One preferred embodiment is illustrated in Figure 3. In this embodiment, the lateral flow chromatography strip (C) is disposed within a housing (H) that acts as a handle for manipulating the device. The housing/handle (H) is provided with viewports (18) for visualizing sample sufficiency and assay results. The capillary matrix protrudes out from the housing to provide a planar surface for insertion into the oral cavity where the capillary matrix face (3) can be contacted to the oral mucosa.

For convenience, the assay device can be optionally equipped with a cover (22) for protection of the capillary matrix surface before and after use. This prevents contamination of the assay device and facilitates sanitary disposal of used devices.

The reader will understand that with the interaction of hydrophilic capillary matrix W and lateral chromatographic strip C, an extremely simple construction is possible. Further, the end product operates in a manner not unlike a conventional thermometer. The test is fast - with prototypes exhibiting about 2 minutes for complete test results. Further, the test easily lends itself to saliva stimulation - such as by place slightly acidic compounds at the end of the wick.

Most importantly, the test only requires minimal volumes of fluid from the oral cavity to run a test. For example in the designs shown, the assays utilized only 100 to 200 µL of oral fluid. This is an improvement of at least a factor of 4 over the sample volume requirements of previous assays.

### Use of the assay device.

In use, the assay device of this invention is inserted into the oral cavity of a mammal (*e.g.* a human) such that the handle within which is disposed the chromatography strip is outside the mouth. The capillary matrix face is preferably contacted to the oral mucosa and, in a particularly preferred embodiment, is pressed to the oral mucosa at the gingival crest *e.g*. pressed between the cheek and the gums.

The assay device is kept in place, preferably without mastication, until sufficient sample is collected. This can be for a predetermined time interval or until the capillary matrix achieves a characteristic tactile quality, or until a sample-sufficiency indicator (*e.g*. a color change) indicates an adequate sample.

At the recommended time (after completion of the immunochromatography assay), the device is read (*e.g*., by visual inspection of the indicator zone(s) through the viewport(s)), to determine whether the subject is positive or negative for the analyte(s) of interest or to quantify the analyte(s).

### Kits for the detection of analytes in oral fluids

In another embodiment, this invention provides kits for the detection of one or more analytes in oral fluids. The kits include one or more of the assay devices described herein. In addition, the kits may include instructional materials containing directions (i.e., protocols) for the practice of the assay methods of this invention. While the instructional materials typically comprise written or printed materials they are not limited to such. Any medium capable of storing such instructions and communicating them to an end user is contemplated by this invention. Such media include, but are not limited to electronic storage media (e.g., magnetic discs, tapes, cartridges, chips), optical media (e.g., CD ROM), and the like. Such media may include addresses to internet sites that provide such instructional materials.

The kits may optionally contain any of the buffers, reagents, detection reagents, and so forth that are useful for the practice of the methods of this invention.

## Claims

1. An apparatus for collection and lateral flow chromatography of an oral fluid, said apparatus comprising:
a capillary matrix having exposed a surface for receiving oral fluid;
a lateral flow chromatography strip where said lateral flow chromatography strip is in communication with said capillary matrix such that when said capillary matrix receives oral fluid, said capillary matrix wicks up said oral fluid and delivers said oral fluid to a receiving area of said lateral flow chromatography strip; and a substantially planar blocking strip placed between the capillary matrix and the lateral flow chromatographic strip.

2. The apparatus of claim 1, wherein saturation of said capillary matrix with an oral fluid does not substantially alter the morphology of said capillary matrix.

3. The apparatus of claim 2, wherein saturation of said capillary matrix with an oral fluid does not substantially alter the average pore size of said capillary matrix.

4. The apparatus of claim 2, wherein saturation of said capillary matrix with an oral fluid does not substantially alter the void volume of said capillary matrix.

5. The apparatus of claim 2, wherein said capillary matrix has an average pore size ranging from about 40 µm to about 250 µm.

6. The apparatus of claim 2, wherein said capillary matrix has a void volume of less than about 60%/cm³.

7. The apparatus of claim 1, wherein said capillary matrix comprises a plastic.

8. The apparatus of claim 7, wherein said capillary matrix comprises a plastic selected from the group consisting of a high density polyethylene (HDPE), an ultra-high molecular weight polyethylene (UHMW), a polypropylene (PP), a polyvinylidene fluoride (PVDF), a polytetrafluoroethylene (PTFE), a nylon 6 (N6), and a polyethersulfone (PES).

9. The apparatus of claim 7, wherein said plastic is hydrophilic or treated to be hydrophilic.

10. The apparatus of claim 1, wherein said capillary matrix, when contacted to an oral mucosa takes up oral fluid from said oral cavity and releases said oral fluid to said receiving area of said lateral flow chromatography strip in under about 1 minute.

11. The apparatus of claim 10, wherein said capillary matrix, when contacted to an oral mucosa takes up oral fluid from said oral cavity and releases said oral fluid to said receiving area of said lateral flow chromatography strip in under about 30 seconds.

12. The apparatus of claim 10, wherein said capillary matrix, is saturated with oral fluid in under about 1 minute.

13. The apparatus of claim 1, wherein said capillary matrix is saturated by less than about 500 µL.

14. The apparatus of claim 1, wherein said capillary matrix releases said oral fluid to said receiving area of said lateral flow chromatography strip without compression of said capillary matrix.

15. The apparatus of claim 14, wherein sufficient oral fluid is released to saturate said receiving area.

16. The apparatus of claim 1, wherein said blocking strip comprises a buffer.

17. The apparatus of claim 1, wherein said blocking strip prevents backflow of reagents from said lateral flow chromatography strip to said capillary matrix.

18. The apparatus of claim 1, further comprising:
a housing having a cavity, wherein said lateral flow chromatography strip extends into the cavity along the housing to an inspection site on the housing; and
at least one inspection site from an exterior of the housing to the lateral chromatographic strip to enable visual inspection of reagents at selected sites on the lateral chromatographic strip.

19. The apparatus of claim 18, wherein said housing acts as a handle for inserting said capillary matrix into said oral cavity.

20. A method of detection or quantifying one or more analytes in an oral fluid, said method comprising the steps of:
i) inserting the apparatus of claim 1 into the oral cavity of a mammal such that said capillary matrix is contacted with an oral mucosal surface whereby said capillary matrix wicks up oral fluid and delivers said oral fluid to a receiving area of said lateral flow chromatography strip; and
ii) reading a signal on said lateral flow chromatography strip that indicates the presence absence or quantity of said one or more analytes.

21. The method of claim 20, wherein saturation of said capillary matrix with an oral fluid does not substantially alter the morphology of said capillary matrix.

22. The method of claim 21, wherein saturation of said capillary matrix with an oral fluid does not substantially alter the average pore site of said capillary matrix.

23. The method of claim 21, wherein saturation of said capillary matrix with an oral fluid does not substantially alter the void volume of said capillary matrix.

24. The method of claim 21, wherein said capillary matrix has an average pore size ranging from about 40 µm to about 250 µm.

25. The method of claim 21, wherein said capillary matrix has a void volume of less than about 60%/cm³.

26. The method of claim 20, wherein said capillary matrix comprises a plastic.

27. The method of claim 26, wherein said capillary matrix comprises a plastic selected from the group consisting of a high density polyethylene (HDPE), an ultra-high molecular weight polyethylene (UHMW), a polypropylene (PP), a polyvinylidene fluoride (PVDF), a polytetrafluoroethylene (PTFE), a nylon 6 (N6), and a polyethersulfone (PES).

28. The method of claim 26, wherein said plastic is hydrophilic or treated to be hydrophilic.

29. The method of claim 20, wherein said capillary matrix, when contacted to an oral mucosa takes up oral fluid from said oral cavity and releases said oral fluid to said receiving area of said lateral flow chromatography strip in under about 1 minute.

30. The method of claim 29, wherein said said capillary matrix, when contacted to an oral mucosa takes up oral fluid from said oral cavity and delivers about 100 µL to about 200 µL of oral fluid to said lateral flow chromatography strip in under about 1 minute.

31. The method of claim 29, wherein said capillary matrix, when contacted to an oral mucosa takes up oral fluid from said oral cavity and releases said oral fluid to said receiving area of said lateral flow chromatography strip in under about 30 seconds.

32. The method of claim 29, wherein said capillary matrix, is saturated with oral fluid in under about 1 minute.

33. The method of claim 20, wherein said capillary matrix is saturated by less than about 500 µL.

34. The method of claim 20, wherein said capillary matrix releases said oral fluid to said receiving area of said lateral flow chromatography strip without compression of said capillary matrix.

35. The method of claim 34, wherein sufficient oral fluid is released to saturate said receiving area.

36. The method of claim 20, wherein said blocking strip further comprises a buffer.

37. The method of claim 20, wherein said blocking strip prevents backflow of reagents from said lateral flow chromatography strip to said capillary matrix.

38. The method of claim 20, wherein said apparatus further comprises:
a housing having a cavity, wherein said lateral flow chromatography strip extends into the cavity along the housing to an inspection site on the housing; and
at least one inspection site from an exterior of the housing to the lateral chromatographic strip to enable visual inspection of reagents at selected sites on the lateral chromatographic strip.

39. The method of claim 38, wherein said housing acts as a handle for inserting said capillary matrix into said oral cavity.

40. A kit for the detection of an analyte in an oral fluid, said kit comprising:
the apparatus of claim 1 for collection and lateral flow chromatography of an oral fluid ; and
instructional materials for said apparatus.

41. The apparatus of claim 1, further comprising:
a housing;
a cavity in the housing,
wherein the lateral chromatography strip extends into the cavity from the cavity along the housing to an inspection site on the housing, the lateral chromatography strip having reagents for binding test analytes;
at least one inspection site from an exterior of the housing to the lateral chromatographic strip to enable visual inspection of reagents at selected sites on the lateral chromatographic strip;
wherein the capillary matrix communicates from the housing to the oral cavity at one end and having communication to the lateral chromatographic strip at the other end, and wherein the capillary matrix is hydrophilic.

42. The apparatus of claim 41, further comprising:
the hydrophilic capillary matrix defining a matrix of channels from material having spherical particles.

43. The apparatus of claim 41 further comprising:
the hydrophilic capillary matrix defining a matrix of channels from material selected from the group including plastic polymer and polystyrene.

44. The apparatus of claim 41, wherein the hydrophilic capillary matrix does not increase its volume during transport of oral fluid.

45. A process of transporting test analytes in oral fluid from an oral cavity to a lateral chromatographic strip comprising the steps of:
providing a lateral chromatographic strip;
providing a housing with a cavity in the housing;
providing a lateral chromatography strip extending into the cavity along the housing to an inspection site on the housing;
providing a hydrophilic capillary matrix communicating from the housing to the oral cavity at one end and having communication to the lateral chromatographic strip at the other end; providing a substantially planar blocking strip between the capillary matrix and the lateral flow chromatographic strip ;
communicating the hydrophilic capillary matrix at the one end to the mouth of a person to be tested; and,
observing the lateral chromatographic strip for inspection of reagents at the selected sites on the lateral chromatographic strip.

46. A process of transporting aqueous fluid from the oral cavity to a lateral chromatographic strip according to claim 45 and comprising the further step of:
providing at least one control site from the exterior of the housing to the lateral chromatographic strip to indicate presence of a minima of fluid to be sampled received from the absorbent pad to the lateral chromatographic strip.

47. A process of transporting aqueous fluid from the oral cavity to a lateral chromatographic strip according to claim 45 and comprising the further step of:
hydrophilic capillary matrix defining a matrix of channels from material having spherical particles.

48. A process of transporting aqueous fluid from the oral cavity to a lateral chromatographic strip according to claim 45 and comprising the further step of:
hydrophilic capillary matrix defining a matrix of channels from material selected from the group including plastic polymer and polystyrene

49. A process of transporting aqueous fluid from the oral cavity to a lateral chromatographic strip according to claim 45 and comprising the further step of:
the provided hydrophilic capillary matrix does not increase its volume during transport of aqueous fluid.

50. The apparatus of claim 1, further comprising a conjugate strip placed between said capillary matrix and said lateral flow chromatographic strip.

51. The apparatus of claim 50, wherein chromatography reagents are disposed in said conjugate strip.

52. The apparatus of claim 1, wherein said blocking strip comprises a detergent.

53. The apparatus of claim 1, wherein said blocking strip comprises a blocking reagent.

54. The apparatus of claim 53, wherein said blocking reagent is selected from the group consisting of: bovine serum albumin (BSA), deoxycholate, and n-lauroyl sarcosine. ;

55. The method of claim 20, wherein said blocking strip comprises a detergent.

56. The method of claim 20, wherein said blocking strip comprises a blockirig reagent.

57. The method of claim 56, wherein said blocking reagents is selected from the group consisting of: bovine serum albumin (BSA), deoxycholate, and n-lauroyl sarcosme

58. The process of claim 45, wherein said blocking strip comprises a detergent.

59. The apparatus of claim 45, wherein said blocking strip comprises a blocking reagent.

60. The apparatus of claim 59, wherein said blocking reagent is selected from the group consisting of: bovine serum albumin (BSA), deoxycholate, and n-lauroyl sarcosine.

61. The apparatus of claim 1, wherein said lateral flow chromatographic strip contains at least one reagent.

62. The apparatus of claim 61; wherein said reagent is a detectable label.

63. The apparatus of claim 62, wherein said detectable label is selected from the group consisting of: magnetic beads, fluorescent dyes, enzymes, and colorimetric labels.

64. The apparatus of claim 63; wherein said fluorescent dyes are selected from the group consisting of: fluorescein isothiocyanate, texas red, rhodamine, green fluorescent protein.

65. The apparatus of claim 63; wherein one of said colorimetric labels is colloidal gold.

66. The apparatus of claim 20 , wherein said analytes are antibodies to selected from the group consisting of: HIV antibodies, HTLV antibodies, *Helicobacter pylori* antibodies, hepatitis antibodies, mumps antibodies, rubella antibodies, cotinine, cocaine, benzoylecgonine, benzodizazpine, tetrahydrocannabinol, nicotine, ethanol theophylline, phenytoin, acetaminophen, lithium, diazepam, nortrypyline, secobarbital, phenobarbitol, theophylline, testosterone, estradiol, 17-hydroxyprogesterone, progesterone, thyroxine, thyroid stimulating hormone, follicle stimulating hormone, luteinizing hormone, transforming growth factor alpba, epidermal growth factor, insulin-like growth factor I and II, growth hormone release inhibiting factor, IGA, glucose, cholesterol, caffeine, and corticosteroid binding globulin.

67. The apparatus of claim 41 wherein at least one of said reagents is a detectable label.

68. The apparatus of claim 67, wherein said detectable label is selected from the group consisting of : magnetic beads, fluorescent dyes, enzymes; and colorimetric labels.

69. The apparatus of claim 68, wherein said fluorescent dyes are selected from the group consisting of: fluorescein isothiocyanate, texas red, rhodamine, green fluorescent protein.

70. The apparatus of claim 68, wherein one of said colorimetric labels is colloidal gold.

71. The apparatus of claim 41, wherein said analytes are antibodies to selected from the group consisting of: HIV antibodies, HTLV antibodies, *Helicobacter pylori* antibodies, hepatitis antibodies, mumps antibodies, rubella antibodies, cotinine, cocaine, benzoylecgonine, benzodizazpine, tetrahydrocannabinol, nicotine, ethanol theophylline, phenytoin, acetaminophen, lithium, diazopam, nortrypyline, secobarbital, phenobarbitol, theophylline, testosterone, estradiol, 17-hydroxyprogesterone, progesterone, thyroxine, thyroid stimulating hormone, follicle stimulating hormone, luteinizing hormone, transforming growth factor alpha, epidermal growth factor, insulin-like growth factor I and II, growth hormone release inhibiting factor, IGA, glucose, cholesterol, caffeine, and corticosteroid binding globulin.

72. A device for collecting and assay of fluids, comprising:
a housing having a fluid receiving end and an assay portion;
a lateral flow assay strip contained substantially within the assay portion, the assay strip containing at least one reagent that is used to detect one of the presence and absence of at least one analyte in a fluid; and
a collection strip for transporting the fluid from a fluid source to the assay strip, the collection strip including:
a first, narrow end contained within the housing and in fluid communication with the assay strip,
a second, enlarged end protruding from the fluid receiving end; and
a blocking strip coupled between and in flow communication with the lateral flow assay strip and the collection strip.

73. The device of claim 72, wherein the collection strip comprises a capillary matrix adapted for rapid wicking of fluid from a fluid source to the assay strip.

74. The device of claim 72, wherein the fluid source is an oral cavity.

75. The device of claim 72, wherein the second end is one of a paddle-shape and substantially bulbous shape.

76. A device for assay of oral fluid, comprising:
an assay portion housing a lateral flow assay strip, the assay strip containing at least one reagent that is used to detect one of the presence and absence of at least one analyte in a fluid; and
a neck portion extending from the assay portion, the neck portion forming a channel for delivery of fluid to the assay strip, the channel being defined by a first, narrow part proximal to the assay portion and a second part including an opening for receiving the oral fluid, wherein the second part includes a channel width that is substantially wider than the channel width at the narrow end;
a collection strip in fluid communication with the lateral assay strip, the collection strip having a first portion disposed within the channel and a second portion protruding outwardly from the neck portion opening; and
a blocking strip coupled between and in flow communication with the lateral flow assay strip and the collection strip.

77. The device of claim 76, wherein the wicking member second portion is paddle shaped.

78. The device of claim 76, wherein the width of the neck portion tapers from the opening width to the narrow end width.

79. The device of claim 72, wherein the assay strip is an immunochromatography strip.

80. The device of claim 72, further comprising a sample adequacy indicator.

81. The device of claim 72, wherein the reagent is an immunospecific binding partner which bears a detectable label.

82. The device of claim 76, wherein the reagent is an enzyme labeled binding partner.

83. The device of claim 72, wherein the reagent comprises one of an antigen and an antibody.

## Patentansprüche

1. Vorrichtung zum Auffangen und zur Lateral-Flow-Chromatographie einer Mundflüssigkeit, wobei die Vorrichtung aufweist:
eine Kapillarmatrix mit einer freiliegenden Oberfläche zum Aufnehmen von Mundflüssigkeit;
einem Lateral-Flow-Chromatographiestreifen, wobei der Lateral-Flow-Chromatographiestreifen mit der Kapillarmatrix in Verbindung steht, so daß beim Aufnehmen von Mundflüssigkeit durch die Kapillarmatrix die Kapillarmatrix die Mundflüssigkeit aufsaugt und die Mundflüssigkeit zu einer Aufnahmefläche des Lateral-Flow-Chromatographiestreifens abgibt; und
einen im wesentlichen ebenen Blockierungsstreifen, der zwischen der Kapillarmatrix und dem Lateral-Flow-Chromatographiestreifen plaziert ist.

2. Vorrichtung nach Anspruch 1, wobei Sättigung der Kapillarmatrix mit einer Mundflüssigkeit die Morphologie der Kapillarmatrix nicht wesentlich ändert.

3. Vorrichtung nach Anspruch 2, wobei Sättigung der Kapillarmatrix mit einer Mundflüssigkeit die mittlere Porengröße der Kapillarmatrix nicht wesentlich ändert.

4. Vorrichtung nach Anspruch 2, wobei Sättigung der Kapillarmatrix mit einer Mundflüssigkeit das Hohlraumvolumen der Kapillarmatrix nicht wesentlich ändert.

5. Vorrichtung nach Anspruch 2, wobei die Kapillarmatrix eine mittlere Porengröße im Bereich von etwa 40 µm bis etwa 250 µm hat.

6. Vorrichtung nach Anspruch 2, wobei die Kapillarmatrix ein Hohlraumvolumen unter etwa 60 %/cm³ hat.

7. Vorrichtung nach Anspruch 1, wobei die Kapillarmatrix einen Kunststoff aufweist.

8. Vorrichtung nach Anspruch 7, wobei die Kapillarmatrix einen Kunststoff aufweist, der aus der Gruppe ausgewählt ist, die aus einem Polyethylen hoher Dichte (HDPE), einem ultrahochmolekularen Polyethylen (UHMW), einem Polypropylen (PP), einem Polyvinylidenfluorid (PVDF), einem Polytetrafluorethylen (PTFE), einem Nylon 6 (N6) und einem Polyethersulfon (PES) besteht.

9. Vorrichtung nach Anspruch 7, wobei der Kunststoff hydrophil oder so behandelt ist, daß er hydrophil ist.

10. Vorrichtung nach Anspruch 1, wobei die Kapillarmatrix bei Kontakt mit einer Mundschleimhaut Mundflüssigkeit aus der Mundhöhle aufnimmt und die Mundflüssigkeit zur Aufnahmefläche des Lateral-Flow-Chromatographiestreifens in weniger als etwa 1 Minute freigibt.

11. Vorrichtung nach Anspruch 10, wobei die Kapillarmatrix bei Kontakt mit einer Mundschleimhaut Mundflüssigkeit aus der Mundhöhle aufnimmt und die Mundflüssigkeit zur Aufnahmefläche des Lateral-Flow-Chromatographiestreifens in weniger als etwa 30 Sekunden freigibt.

12. Vorrichtung nach Anspruch 10, wobei die Kapillarmatrix mit Mundflüssigkeit in weniger als etwa 1 Minute gesättigt wird.

13. Vorrichtung nach Anspruch 1, wobei die Kapillarmatrix durch weniger als etwa 500 µl gesättigt wird.

14. Vorrichtung nach Anspruch 1, wobei die Kapillarmatrix die Mundflüssigkeit zur Aufnahmefläche des Lateral-Flow-Chromatographiestreifens ohne Kompression der Kapillarmatrix freigibt.

15. Vorrichtung nach Anspruch 14, wobei ausreichende Mundflüssigkeit freigegeben wird, um die Aufnahmefläche zu sättigen.

16. Vorrichtung nach Anspruch 1, wobei der Blockierungsstreifen einen Puffer aufweist.

17. Vorrichtung nach Anspruch 1, wobei der Blockierungsstreifen Rückfluß von Reagenzien vom Lateral-Flow-Chromatographiestreifen zur Kapillarmatrix verhindert.

18. Vorrichtung nach Anspruch 1, ferner mit:
einem Gehäuse mit einem Hohlraum, wobei sich der Lateral-Flow-Chromatographiestreifen in den Hohlraum entlang dem Gehäuse zu einer Kontrollstelle am Gehäuse erstreckt; und
mindestens einer Kontrollstelle von einer Außenseite des Gehäuses zum Lateral-Flow-Chromatographiestreifen, um Sichtkontrolle von Reagenzien an ausgewählten Stellen auf dem Lateral-Flow-Chromatographiestreifen zu ermöglichen.

19. Vorrichtung nach Anspruch 18, wobei das Gehäuse als Griff zum Einführen der Kapillarmatrix in die Mundhöhle wirkt.

20. Verfahren zum Detektieren oder Quantifizieren eines oder mehrerer Analyte in einer Mundflüssigkeit, wobei das Verfahren die folgenden Schritte aufweist:
i) Einführen der Vorrichtung nach Anspruch 1 in die Mundhöhle eines Säugers, so daß die Kapillarmatrix mit einer Mundschleimhautoberfläche in Kontakt kommt, wodurch die Kapillarmatrix Mundflüssigkeit aufsaugt und die Mundflüssigkeit zu einer Aufnahmefläche des Lateral-Flow-Chromatographiestreifens abgibt; und
ii) Ablesen eines Signals auf dem Lateral-Flow-Chromatographiestreifen, das das Vorhandensein, das Fehlen oder die Menge des einen oder der mehreren Analyte anzeigt.

21. Verfahren nach Anspruch 20, wobei Sättigung der Kapillarmatrix mit einer Mundflüssigkeit die Morphologie der Kapillarmatrix nicht wesentlich ändert.

22. Verfahren nach Anspruch 21, wobei Sättigung der Kapillarmatrix mit einer Mundflüssigkeit die mittlere Porengröße der Kapillarmatrix nicht wesentlich ändert.

23. Verfahren nach Anspruch 21, wobei Sättigung der Kapillarmatrix mit einer Mundflüssigkeit das Hohlraumvolumen der Kapillarmatrix nicht wesentlich ändert.

24. Verfahren nach Anspruch 21, wobei die Kapillarmatrix eine mittlere Porengröße im Bereich von etwa 40 µm bis etwa 250 µm hat.

25. Verfahren nach Anspruch 21, wobei die Kapillarmatrix ein Hohlraumvolumen unter etwa 60 %/cm³ hat.

26. Verfahren nach Anspruch 20, wobei die Kapillarmatrix einen Kunststoff aufweist.

27. Verfahren nach Anspruch 26, wobei die Kapillarmatrix einen Kunststoff aufweist, der aus der Gruppe ausgewählt ist, die aus einem Polyethylen hoher Dichte (HDPE), einem ultrahochmolekularen Polyethylen (UHMW), einem Polypropylen (PP), einem Polyvinylidenfluorid (PVDF), einem Polytetrafluorethylen (PTFE), einem Nylon 6 (N6) und einem Polyethersulfon (PES) besteht.

28. Verfahren nach Anspruch 26, wobei der Kunststoff hydrophil oder so behandelt ist, daß er hydrophil ist.

29. Verfahren nach Anspruch 20, wobei die Kapillarmatrix bei Kontakt mit einer Mundschleimhaut Mundflüssigkeit aus der Mundhöhle aufnimmt und die Mundflüssigkeit zur Aufnahmefläche des Lateral-Flow-Chromatographiestreifens in weniger als etwa 1 Minute freigibt.

30. Verfahren nach Anspruch 29, wobei die Kapillarmatrix bei Kontakt mit einer Mundschleimhaut Mundflüssigkeit aus der Mundhöhle aufnimmt und etwa 100 µl bis etwa 200 µl Mundflüssigkeit zum Lateral-Flow-Chromatographiestreifen in weniger als etwa 1 Minute abgibt.

31. Verfahren nach Anspruch 29, wobei die Kapillarmatrix bei Kontakt mit einer Mundschleimhaut Mundflüssigkeit aus der Mundhöhle aufnimmt und die Mundflüssigkeit zur Aufnahmefläche des Lateral-Flow-Chromatographiestreifens in weniger als etwa 30 Sekunden freigibt.

32. Verfahren nach Anspruch 29, wobei die Kapillarmatrix mit Mundflüssigkeit in weniger als etwa 1 Minute gesättigt wird.

33. Verfahren nach Anspruch 20, wobei die Kapillarmatrix durch weniger als etwa 500 µl gesättigt wird.

34. Verfahren nach Anspruch 20, wobei die Kapillarmatrix die Mundflüssigkeit zur Aufnahmefläche des Lateral-Flow-Chromatographiestreifens ohne Kompression der Kapillarmatrix freigibt.

35. Verfahren nach Anspruch 34, wobei ausreichende Mundflüssigkeit freigegeben wird, um die Aufnahmefläche zu sättigen.

36. Verfahren nach Anspruch 20, wobei der Blockierungsstreifen ferner einen Puffer aufweist.

37. Verfahren nach Anspruch 20, wobei der Blockierungsstreifen Rückfluß von Reagenzien vom Lateral-Flow-Chromatographiestreifen zur Kapillarmatrix verhindert.

38. Verfahren nach Anspruch 20, wobei die Vorrichtung ferner aufweist:
ein Gehäuse mit einem Hohlraum, wobei sich der Lateral-Flow-Chromatographiestreifen in den Hohlraum entlang dem Gehäuse zu einer Kontrollstelle am Gehäuse erstreckt; und
mindestens eine Kontrollstelle von einer Außenseite des Gehäuses zum Lateral-Flow-Chromatographiestreifen, um Sichtkontrolle von Reagenzien an ausgewählten Stellen auf dem Lateral-Flow-Chromatographiestreifen zu ermöglichen.

39. Verfahren nach Anspruch 38, wobei das Gehäuse als Griff zum Einführen der Kapillarmatrix in die Mundhöhle wirkt.

40. Ausrüstungssatz zur Detektion eines Analyten in einer Mundflüssigkeit, wobei der Ausrüstungssatz aufweist:
die Vorrichtung nach Anspruch 1 zum Auffangen und zur Lateral-Flow-Chromatographie einer Mundflüssigkeit; und Bedienungsanleitungen für die Vorrichtung.

41. Vorrichtung nach Anspruch 1, ferner mit:
einem Gehäuse;
einem Hohlraum im Gehäuse, wobei sich der Lateral-Flow-Chromatographiestreifen in den Hohlraum vom Hohlraum entlang dem Gehäuse zu einer Kontrollstelle am Gehäuse erstreckt, wobei der Lateral-Flow-Chromatographiestreifen Reagenzien zum Binden von Testanalyten hat;
mindestens einer Kontrollstelle von einer Außenseite des Gehäuses zum Lateral-Flow-Chromatographiestreifen, um Sichtkontrolle von Reagenzien an ausgewählten Stellen auf dem Lateral-Flow-Chromatographiestreifen zu ermöglichen;
wobei die Kapillarmatrix vom Gehäuse zur Mundhöhle an einem Ende kommuniziert und eine Verbindung zum Lateral-Flow-Chromatographiestreifen am anderen Ende hat und wobei die Kapillarmatrix hydrophil ist.

42. Vorrichtung nach Anspruch 41, ferner mit:
der hydrophilen Kapillarmatrix, die eine Matrix von Kanälen aus Material mit kugelförmigen Teilchen bildet.

43. Vorrichtung nach Anspruch 41, ferner mit:
der hydrophilen Kapillarmatrix, die eine Matrix von Kanälen aus Material bildet, das aus der Gruppe ausgewählt ist, die Polymer- und Polystyrolkunststoff aufweist.

44. Vorrichtung nach Anspruch 41, wobei die hydrophile Kapillarmatrix ihr Volumen beim Transport von Mundflüssigkeit nicht erhöht.

45. Prozeß zum Transportieren von Testanalyten in Mundflüssigkeit von einer Mundhöhle zu einem Lateral-Flow-Chromatographiestreifen mit den folgenden Schritten:
Bereitstellen eines Lateral-Flow-Chromatographiestreifens;
Bereitstellen eines Gehäuses mit einem Hohlraum im Gehäuse;
Bereitstellen eines Lateral-Flow-Chromatographiestreifens, der sich in den Hohlraum entlang dem Gehäuse zu einer Kontrollstelle am Gehäuse erstreckt;
Bereitstellen einer hydrophilen Kapillarmatrix, die vom Gehäuse zur Mundhöhle an einem Ende kommuniziert und eine Verbindung zum Lateral-Flow-Chromatographiestreifen am anderen Ende hat;
Bereitstellen eines im wesentlichen ebenen Blockierungsstreifens zwischen der Kapillarmatrix und dem Lateral-Flow-Chromatographiestreifen;
Herstellen einer Verbindung der hydrophilen Kapillarmatrix an dem einen Ende mit dem Mund einer zu testenden Person; und
Beobachten des Lateral-Flow-Chromatographiestreifens zur Kontrolle von Reagenzien an den ausgewählten Stellen auf dem Lateral-Flow-Chromatographiestreifen.

46. Prozeß zum Transportieren wäßriger Flüssigkeit aus der Mundhöhle zu einem Lateral-Flow-Chromatographiestreifen nach Anspruch 45 und ferner mit dem folgenden Schritt:
Bereitstellen mindestens einer Kontrollstelle von der Außenseite des Gehäuses zum Lateral-Flow-Chromatographiestreifen, um Vorhandensein eines Minimums zu beprobender Flüssigkeit anzuzeigen, die vom absorbierenden Kissen zum Lateral-Flow-Chromatographiestreifen aufgenommen wurde.

47. Prozeß zum Transportieren wäßriger Flüssigkeit aus der Mundhöhle zu einem Lateral-Flow-Chromatographiestreifen nach Anspruch 45 und ferner mit dem folgenden Schritt:
einer hydrophilen Kapillarmatrix, die eine Matrix von Kanälen aus Material mit kugelförmigen Teilchen bildet.

48. Prozeß zum Transportieren wäßriger Flüssigkeit aus der Mundhöhle zu einem Lateral-Flow-Chromatographiestreifen nach Anspruch 45 und ferner mit dem folgenden Schritt:
einer hydrophilen Kapillarmatrix, die eine Matrix von Kanälen aus Material bildet, das aus der Gruppe ausgewählt ist, die Polymer- und Polystyrolkunststoff aufweist.

49. Prozeß zum Transportieren wäßriger Flüssigkeit aus der Mundhöhle zu einem Lateral-Flow-Chromatographiestreifen nach Anspruch 45 und ferner mit dem folgenden Schritt:
die bereitgestellte hydrophile Kapillarmatrix erhöht nicht ihr Volumen beim Transport von wäßriger Flüssigkeit.

50. Vorrichtung nach Anspruch 1, ferner mit einem Konjugatstreifen, der zwischen der Kapillarmatrix und dem Lateral-Flow-Chromatographiestreifen plaziert ist.

51. Vorrichtung nach Anspruch 50, wobei Chromatographiereagenzien im Konjugatstreifen angeordnet sind.

52. Vorrichtung nach Anspruch 1, wobei der Blockierungsstreifen ein Detergens aufweist.

53. Vorrichtung nach Anspruch 1, wobei der Blockierungsstreifen ein Blockierungsreagens aufweist.

54. Vorrichtung nach Anspruch 53, wobei das Blockierungsreagens aus der Gruppe ausgewählt ist, die aus bovinem Serumalbumin (BSA), Desoxycholat und n-Lauroyl-Sarkosin besteht.

55. Verfahren nach Anspruch 20, wobei der Blockierungsstreifen ein Detergens aufweist.

56. Verfahren nach Anspruch 20, wobei der Blockierungsstreifen ein Blockierungsreagens aufweist.

57. Verfahren nach Anspruch 56, wobei das Blockierungsreagens aus der Gruppe ausgewählt ist, die aus bovinem Serumalbumin (BSA), Desoxycholat und n-Lauroyl-Sarkosin besteht.

58. Prozeß nach Anspruch 45, wobei der Blockierungsstreifen ein Detergens aufweist.

59. Vorrichtung nach Anspruch 45, wobei der Blockierungsstreifen ein Blockierungsreagens aufweist.

60. Vorrichtung nach Anspruch 59, wobei das Blockierungsreagens aus der Gruppe ausgewählt ist, die aus bovinem Serumalbumin (BSA), Desoxycholat und n-Lauroyl-Sarkosin besteht.

61. Vorrichtung nach Anspruch 1, wobei der Lateral-Flow-Chromatographiestreifen mindestens ein Reagens enthält.

62. Vorrichtung nach Anspruch 61, wobei das Reagens ein detektierbares Label ist.

63. Vorrichtung nach Anspruch 62, wobei das detektierbare Label aus der Gruppe ausgewählt ist, die aus Magnetkügelchen, Fluoreszenzfarbstoffen, Enzymen und kolorimetrischen Labels besteht.

64. Vorrichtung nach Anspruch 63, wobei die Fluoreszenzfarbstoffe aus der Gruppe ausgewählt sind, die aus Fluoresceinisothiocyanat, Texasrot, Rhodamin, grün fluoreszierendes Protein besteht.

65. Vorrichtung nach Anspruch 63, wobei eines der kolorimetrischen Labels Kolloidgold ist.

66. Vorrichtung nach Anspruch 20, wobei die Analyte Antikörper sind, die aus der Gruppe ausgewählt sind, die aus folgendem besteht: HIV-Antikörper, HTLV-Antikörper, Helicobacter-pylori-Antikörper, Hepatitisantikörper, Mumpsantikörper, Rötelnantikörper, Cotinin, Cocain, Benzoylecgonin, Benzodiazepin, Tetrahydrocannabinol, Nicotin, Ethanoltheophyllin, Phenytoin, Acetaminophen, Lithium, Diazepam, Nortrypylin, Secobarbital, Phenobarbitol, Theophyllin, Testosteron, Estradiol, 17-Hydroxyprogesteron, Progesteron, Thyroxin, thyroid-stimulierendes Hormon, follikelstimulierendes Hormon, luteinisierendes Hormon, von transformierten Zellen gebildeter Wachstumsfaktor Alpha, epidermaler Wachstumsfaktor, insulin-ähnlicher Wachstumsfaktor I und II, wachstumshemmendes Hormon, IGA, Glucose, Cholesterol, Caffein und corticosteroidbindendes Globulin.

67. Vorrichtung nach Anspruch 41, wobei mindestens eines der Reagenzien ein detektierbares Label ist.

68. Vorrichtung nach Anspruch 67, wobei das detektierbare Label aus der Gruppe ausgewählt ist, die aus Magnetkügelchen, Fluoreszenzfarbstoffen, Enzymen und kolorimetrischen Labels besteht.

69. Vorrichtung nach Anspruch 68, wobei die Fluoreszenzfarbstoffe aus der Gruppe ausgewählt sind, die aus Fluoresceinisothiocyanat, Texasrot, Rhodamin, grün fluoreszierendes Protein besteht.

70. Vorrichtung nach Anspruch 68, wobei eines der kolorimetrischen Labels Kolloidgold ist.

71. Vorrichtung nach Anspruch 41, wobei die Analyte Antikörper sind, die aus der Gruppe ausgewählt sind, die aus folgendem besteht: HIV-Antikörper, HTLV-Antikörper, *Helicobacter-pylori*-Antikörper, Hepatitisantikörper, Mumpsantikörper, Rötelnantikörper, Cotinin, Cocain, Benzoylecgonin, Benzodiazepin, Tetrahydrocannabinol, Nicotin, Ethanoltheophyllin, Phenytoin, Acetaminophen, Lithium, Diazepam, Nortrypylin, Secobarbital, Phenobarbitol, Theophyllin, Testosteron, Estradiol, 17-Hydroxyprogesteron, Progesteron, Thyroxin, thyroid-stimulierendes Hormon, follikelstimulierendes Hormon, luteinisierendes Hormon, von transformierten Zellen gebildeter Wachstumsfaktor Alpha, epidermaler Wachstumsfaktor, insulin-ähnlicher Wachstumsfaktor I und II, wachstumshemmendes Hormon, IGA, Glucose, Cholesterol, Caffein und corticosteroidbindendes Globulin.

72. Gerät zum Auffangen und Analysieren von Flüssigkeiten mit:
einem Gehäuse, das ein Flüssigkeitsaufnahmeende und einen Analysenabschnitt hat;
einem Lateral-Flow-Analysenstreifen, der im wesentlichen im Analysenabschnitt enthalten ist, wobei der Analysenstreifen mindestens ein Reagens enthält, das verwendet wird, das Vorhandensein oder Fehlen mindestens eines Analyten in einer Flüssigkeit zu detektieren; und
einem Auffangstreifen zum Transportieren der Flüssigkeit von einer Flüssigkeitsquelle zum Analysenstreifen, wobei der Auffangstreifen aufweist:
ein erstes, schmales Ende, das im Gehäuse enthalten ist und in Flüssigkeitsverbindung mit dem Analysenstreifen steht;
ein zweites, vergrößertes Ende, das aus dem Flüssigkeitsaufnahmeende vorsteht; und
einen Blockierungsstreifen, der zwischen dem Lateral-Flow-Analysenstreifen und dem Auffangstreifen gekoppelt ist und mit ihnen in Fließverbindung steht.

73. Gerät nach Anspruch 72, wobei der Auffangstreifen eine Kapillarmatrix aufweist, die zum schnellen Aufsaugen von Flüssigkeit von einer Flüssigkeitsquelle zum Analysenstreifen geeignet ist.

74. Gerät nach Anspruch 72, wobei die Flüssigkeitsquelle eine Mundhöhle ist.

75. Gerät nach Anspruch 72, wobei das zweite Ende paddelförmig oder im wesentlichen knollenförmig ist.

76. Gerät zur Analyse von Mundflüssigkeit mit:
einem Analysenabschnitt, in dem ein Lateral-Flow-Analysenstreifen untergebracht ist, wobei der Analysenstreifen mindestens ein Reagens enthält, das verwendet wird, das Vorhandensein oder Fehlen mindestens eines Analyten in einer Flüssigkeit zu detektieren; und
einem Halsabschnitt, der sich vom Analysenabschnitt erstreckt, wobei der Halsabschnitt einen Kanal zur Abgabe von Flüssigkeit zum Analysenstreifen bildet, wobei der Kanal durch ein erstes, schmales teil proximal zum Analysenabschnitt und ein zweites Teil mit einer Öffnung zum Aufnehmen der Mundflüssigkeit gebildet ist, wobei das zweite Teil eine Kanalbreite aufweist, die wesentlich breiter als die Kanalbreite am schmalen Ende ist;
einem Auffangstreifen in Flüssigkeitsverbindung mit dem Lateral-Flow-Analysenstreifen, wobei der Auffangstreifen einen im Kanal angeordneten ersten Abschnitt und
einen aus der Halsabschnittsöffnung nach außen vorstehenden zweiten Abschnitt hat; und
einem Blockierungsstreifen, der zwischen dem Lateral-Flow-Analysenstreifen und dem Auffangstreifen gekoppelt ist und mit ihnen in Fließverbindung steht.

77. Gerät nach Anspruch 76, wobei der zweite Abschnitt des Aufsaugteils paddelförmig ist.

78. Gerät nach Anspruch 76, wobei sich die Breite des Halsabschnitts von der Öffnungsbreite auf die Breite des schmalen Endes verjüngt.

79. Gerät nach Anspruch 72, wobei der Analysenstreifen ein Immunchromatographiestreifen ist.

80. Gerät nach Anspruch 72, ferner mit einem Probensuffizienzindikator.

81. Gerät nach Anspruch 72, wobei das Reagens ein immunspezifischer Bindungspartner ist, der ein detektierbares Label trägt.

82. Gerät nach Anspruch 76, wobei das Reagens ein enzymmarkierter Bindungspartner ist.

83. Gerät nach Anspruch 72, wobei das Reagens ein Antigen oder einen Antikörper aufweist.

## Revendications

1. Appareil de collecte et d'immunochromatographie sur membrane d'un fluide oral, ledit appareil comprenant :
une matrice capillaire ayant une surface exposée pour recevoir un fluide oral ;
une bande d'immunochromatographie sur membrane où ladite bande d'immunochromatographie sur membrane est en communication avec ladite matrice capillaire de sorte que lorsque ladite matrice capillaire reçoit un fluide oral, ladite matrice capillaire fait monter par capillarité ledit fluide oral et délivre ledit fluide oral à une zone de réception de ladite bande d'immunochromatographie sur membrane ; et
une bande de blocage sensiblement plane placée entre la matrice capillaire et la bande pour immunochromatographie sur membrane.

2. Appareil selon la revendication 1, dans lequel la saturation de ladite matrice capillaire avec un fluide oral ne modifie pas sensiblement la morphologie de ladite matrice capillaire.

3. Appareil selon la revendication 2, dans lequel la saturation de ladite matrice capillaire avec un fluide oral ne modifie pas sensiblement la taille moyenne de pores de ladite matrice capillaire.

4. Appareil selon la revendication 2, dans lequel la saturation de ladite matrice capillaire avec un fluide oral ne modifie pas sensiblement le volume vide de ladite matrice capillaire.

5. Appareil selon la revendication 2, dans lequel ladite matrice capillaire a une taille moyenne de pores variant d'environ 40 µm à environ 250 µm.

6. Appareil selon la revendication 2, dans lequel ladite matrice capillaire a un volume vide inférieur à environ 60%/cm³.

7. Appareil selon la revendication 1, dans lequel ladite matrice capillaire comprend un plastique.

8. Appareil selon la revendication 7, dans lequel ladite matrice capillaire comprend un plastique choisi dans le groupe constitué par un polyéthylène haute densité (HDPE), un polyéthylène de poids moléculaire élevé (UHMW), un polypropylène (PP), un polyfluorure de vinylidène (PVDF), un polytétrafluoroéthylène (PTFE), un nylon 6 (N6) et un polyéthersulfone (PES).

9. Appareil selon la revendication 7, dans lequel ledit plastique est hydrophile ou traité pour être hydrophile.

10. Appareil selon la revendication 1, dans lequel ladite matrice capillaire, quand elle est en contact avec une muqueuse orale reprend le fluide oral de ladite cavité orale et libère ledit fluide oral à ladite zone de réception de ladite bande d'immunochromatographie sur membrane en moins d'environ 1 minute.

11. Appareil selon la revendication 10, dans lequel ladite matrice capillaire, quand elle est en contact avec une muqueuse orale reprend le fluide oral provenant de ladite cavité orale et libère ledit fluide oral à ladite zone de réception de ladite bande d'immunochromatographie sur membrane en moins d'environ 30 secondes.

12. Appareil selon la revendication 10, dans lequel ladite matrice capillaire est saturée de fluide oral en moins d'environ 1 minute.

13. Appareil selon la revendication 1, dans lequel ladite matrice capillaire est saturée par moins d'environ 500 µL.

14. Appareil selon la revendication 1, dans lequel ladite matrice capillaire libère ledit fluide oral sur ladite zone de réception de ladite bande d'immunochromatographie sur membrane sans compression de ladite matrice capillaire.

15. Appareil selon la revendication 14, dans lequel le fluide oral en quantité suffisante est libéré pour saturer ladite zone de réception.

16. Appareil selon la revendication 1, dans lequel ladite bande de blocage comprend un tampon.

17. Appareil selon la revendication 1, dans lequel ladite bande de blocage empêche l'écoulement retour des réactifs provenant de ladite bande d'immunochromatographie sur membrane vers ladite matrice capillaire.

18. Appareil selon la revendication 1, comprenant en outre :
un logement ayant une cavité, dans laquelle ladite bande d'immunochromatographie sur membrane s'étend dans la cavité le long du logement vers un site d'inspection sur le logement ; et
au moins un site d'inspection provenant de l'extérieur du logement vers la bande d'immunochromatographie sur membrane pour permettre une inspection, visuelle des réactifs en des sites choisis sur la bande d'immunochromatographie sur membrane.

19. Appareil selon la revendication 18, dans lequel ledit logement agit comme une poignée pour insérer ladite matrice capillaire dans ladite cavité orale.

20. Procédé de détection ou de quantification d'un ou plusieurs analytes dans un fluide oral, ledit procédé comprenant les étapes consistant à :
i) insérer l'appareil selon la revendication 1 dans la cavité orale d'un mammifère de sorte que ladite matrice capillaire soit en contact avec une surface muqueuse orale de sorte que ladite matrice capillaire fasse monter par capillarité le fluide oral et délivre ledit fluide oral sur une zone de réception de ladite bande d'immunochromatographie sur membrane ; et
ii) lire un signal sur ladite bande d'immunochromatographie sur membrane qui indique la présence, l'absence ou la quantité dudit un ou plusieurs analytes.

21. Procédé selon la revendication 20, dans lequel la saturation de ladite matrice capillaire avec un fluide oral ne modifie pas sensiblement la morphologie de ladite matrice capillaire.

22. Procédé selon la revendication 21, dans lequel la saturation de ladite matrice capillaire avec un fluide oral ne modifie pas sensiblement la taille moyenne de pores de ladite matrice capillaire.

23. Procédé selon la revendication 21, dans lequel la saturation de ladite matrice capillaire avec un fluide oral ne modifie pas sensiblement le volume vide de ladite matrice capillaire.

24. Procédé selon la revendication 21, dans lequel ladite matrice capillaire a une taille moyenne de pores variant d'environ 40 µm à environ 250 µm.

25. Procédé selon la revendication 21, dans lequel ladite matrice capillaire a un volume vide inférieur à environ 60%/cm³.

26. Procédé selon la revendication 20, dans lequel ladite matrice capillaire comprend un plastique.

27. Procédé selon la revendication 26, dans lequel ladite matrice capillaire comprend un plastique choisi parmi le groupe constitué par un polyéthylène haute densité (HDPE), un polyéthylène de poids moléculaire élevé (UHMW), un polypropylène (PP), un polyfluorure de vinylidène (PVDF), un polytétrafluoroéthylène (PTFE), un nylon 6 (N6) et un polyéthersulfone (PES).

28. Procédé selon la revendication 26, dans lequel ledit plastique est hydrophile ou traité pour être hydrophile.

29. Procédé selon la revendication 20, dans lequel ladite matrice capillaire, quand elle est en contact avec une muqueuse orale reprend le fluide oral de ladite cavité orale et libère ledit fluide oral à ladite zone de réception de ladite bande d'immunochromatographie sur membrane en moins d'environ 1 minute.

30. Procédé selon la revendication 29, dans lequel ladite matrice capillaire, quand elle est en contact avec une muqueuse orale reprend le fluide oral de ladite cavité orale et délivre environ 100 µL à environ 200 µL de fluide oral à ladite bande d'immunochromatographie sur membrane en moins d'environ 1 minute.

31. Procédé selon la revendication 29, dans lequel ladite matrice capillaire, quand elle est en contact avec une muqueuse orale reprend le fluide oral de ladite cavité orale et libère ledit fluide oral à ladite zone de réception de ladite bande d'immunochromatographie sur membrane en moins d'environ 30 secondes.

32. Procédé selon la revendication 29, dans lequel ladite matrice capillaire est saturée avec un fluide oral en moins d'environ 1 minute.

33. Procédé selon la revendication 20, dans lequel ladite matrice capillaire est saturée par moins d'environ 500 *µ*L.

34. Procédé selon la revendication 20, dans lequel ladite matrice capillaire libère ledit fluide oral à ladite zone de réception de ladite bande d'immunochromatographie sur membrane sans compression de ladite matrice capillaire.

35. Procédé selon la revendication 34, dans lequel le fluide oral en quantité suffisante est libéré pour saturer ladite zone de réception.

36. Procédé selon la revendication 20, dans lequel ladite bande de blocage comprend en outre un tampon.

37. Procédé selon la revendication 20, dans lequel ladite bande de blocage empêche le courant de retour des réactifs de ladite bande d'immunochromatographie sur membrane vers ladite matrice capillaire.

38. Procédé selon la revendication 20, dans lequel ledit appareil comprend en outre :
un logement ayant une cavité, dans laquelle ladite bande d'immunochromatographie sur membrane s'étend dans la cavité le long du logement vers un site d'inspection sur le logement ; et
au moins un site d'inspection provenant de l'extérieur du logement vers la bande d'immunochromatographie sur membrane pour permettre une inspection visuelle des réactifs en des sites choisis sur la bande d'immunochromatographie sur membrane.

39. Procédé selon la revendication 38, dans lequel ledit logement agit comme une poignée pour insérer ladite matrice capillaire dans ladite cavité orale.

40. Trousse pour la détection d'un analyte dans un fluide oral, ladite trousse comprenant :
l'appareil selon la revendication 1 pour la récolte et l'immunochromatographie sur membrane d'un fluide oral ; et
des matériaux d'instruction pour ledit appareil.

41. Appareil selon la revendication 1, comprenant en outre :
un logement ;
une cavité dans le logement ; dans laquelle la bande d'immunochromatographie sur membrane s'étend dans la cavité depuis la cavité le long du logement vers un site d'inspection sur le logement, la bande d'immunochromatographie sur membrane ayant des réactifs pour la liaison des analytes test ;
au moins un site d'inspection provenant de l'extérieur du logement vers la bande d'immunochromatographie sur membrane pour permettre une inspection visuelle des réactifs en des sites choisis sur la bande d'immunochromatographie sur membrane ;
dans lequel la matrice capillaire communique depuis le logement vers la cavité orale à une extrémité et a une communication vers la bande d'immunochromatographie sur membrane de l'autre côté, et dans lequel la matrice capillaire est hydrophile.

42. Appareil selon la revendication 41, comprenant en outre :
la matrice capillaire hydrophile définissant une matrice de canaux provenant d'un matériau ayant des particules sphériques.

43. Appareil selon la revendication 41, comprenant en outre :
la matrice capillaire hydrophile définissant une matrice de canaux provenant d'un matériau choisi dans le groupe comprenant un polymère plastique et un polystyrène.

44. Appareil selon la revendication 41, dans lequel la matrice capillaire hydrophile n'augmente pas de volume au cours du transport du fluide oral.

45. Procédé de transport des analytes test dans un fluide oral provenant d'une cavité orale vers une bande d'immunochromatographie sur membrane comprenant les étapes consistant à :
fournir une bande d'immunochromatographie sur membrane ;
fournir un logement avec une cavité dans le logement ;
fournir une bande d'immunochromatographie sur membrane qui s'étend dans la cavité le long du logement vers un site d'inspection sur le logement ;
fournir une matrice capillaire hydrophile communiquant depuis le logement vers la cavité orale à une extrémité et ayant une communication vers la bande d'immunochromatographie sur membrane de l'autre coté ; et
fournir une bande de blocage sensiblement plane entre la matrice capillaire et la bande d'immunochromatographie sur membrane ;
faire communiquer la matrice capillaire hydrophile d'une extrémité avec la bouche d'une personne à tester ; et
observer la bande d'immunochromatographie sur membrane pour l'inspection des réactifs en des sites choisis sur la bande d'immunochromatographie sur membrane.

46. Procédé de transport d'un fluide aqueux provenant de la cavité orale vers une bande d'immunochromatographie sur membrane selon la revendication 45 et comprenant l'étape supplémentaire consistant à :
fournir au moins un site témoin de l'extérieur du logement vers la bande d'immunochromatographie sur membrane pour indiquer la présence d'un minimum de fluide à échantillonner reçu du tampon absorbant vers la bande d'immunochromatographie sur membrane.

47. Procédé de transport d'un fluide aqueux depuis la cavité orale vers une bande d'immunochromatographie sur membrane selon la revendication 45 et comprenant l'étape supplémentaire consistant à :
fournir une matrice capillaire hydrophile définissant une matrice de canaux à partir d'un matériau ayant des particules sphériques.

48. Processus de transport d'un fluide aqueux depuis la cavité orale vers une bande d'immunochromatographie latérale selon la revendication 45 et comprenant l'étape supplémentaire consistant à :
fournir une matrice capillaire hydrophile définissant une matrice de canaux provenant d'un matériau choisi dans le groupe comprenant un polymère plastique et un polystyrène.

49. Procédé de transport d'un fluide aqueux depuis la cavité orale vers une bande d'immunochromatographie sur membrane selon la revendication 45 et comprenant l'étape supplémentaire selon laquelle :
la matrice capillaire hydrophile fournie n'augmente pas de volume au cours du transport d'un fluide aqueux.

50. Appareil selon la revendication 1, comprenant en outre une bande conjuguée placée entre ladite matrice capillaire et ladite bande d'immunochromatographie sur membrane.

51. Appareil selon la revendication 50, dans lequel les réactifs de chromatographie sont disposés dans ladite bande conjuguée.

52. Appareil selon la revendication 1, dans lequel ladite bande de blocage comprend un détergent.

53. Appareil selon la revendication 1, dans lequel ladite bande de blocage comprend un réactif de blocage.

54. Appareil selon la revendication 53, dans lequel ledit réactif de blocage est choisi dans le groupe constitué par la sérum albumine bovine (BSA), le désoxycholate et la n-lauroyl-sarcosine.

55. Procédé selon la revendication 20, dans lequel ladite bande de blocage comprend un détergent.

56. Procédé selon la revendication 20, dans lequel ladite bande de blocage comprend un réactif de blocage.

57. Procédé selon la revendication 56, dans lequel lesdits réactifs de blocage sont choisis dans le groupe constitué par la sérum albumine bovine (BSA), le désoxycholate et la n-lauroyl-sarcosine.

58. Procédé selon la revendication 45, dans lequel ladite bande de blocage comprend un détergent.

59. Appareil selon la revendication 45, dans lequel ladite bande de blocage comprend un réactif de blocage.

60. Appareil selon la revendication 59, dans lequel ledit réactif de blocage est choisi dans le groupe constitué par la sérum albumine bovine (BSA), le désoxycholate et la n-lauroyl-sarcosine.

61. Appareil selon la revendication 1, dans lequel ladite bande d'immunochromatographie sur membrane contient au moins un réactif.

62. Appareil selon la revendication 61, dans lequel ledit réactif est un marqueur détectable.

63. Appareil selon la revendication 62, dans lequel ledit marqueur détectable est choisi dans le groupe constitué par des billes magnétiques, des colorants fluorescents, des enzymes et des marqueurs colorimétriques.

64. Appareil selon la revendication 63, dans lequel lesdits colorants fluorescents sont choisis dans le groupe constitué de : isothiocyanate de fluorescéine, rouge texas, rhodamine, protéine à fluorescence verte.

65. Appareil selon la revendication 63, dans lequel un desdits marqueurs colorimétriques est de l'or colloïdal.

66. Appareil selon la revendication 20, dans lequel lesdits analytes sont des anticorps choisis dans le groupe constitué par : les anticorps VIH, les anticorps HTLV, les anticorps *Helicobacter pylori,* les anticorps de l'hépatite, les anticorps des oreillons, les anticorps de la rubéole, la cotinine, la cocaïne, la benzoylecgonine, la benzodiazépine, le tétrahydrocannabinol, la nicotine, la théophylline éthanol, la phénytoïne, le paracétamol, le lithium, le diazépam, la nortrypyline, le sécobarbital, le phénobarbitol, la théophylline, la testostérone, l'oestradiol, la 17-hydroxyprogestérone, la progestérone, la thyroxine, la thyrolibérine, l'hormone follicostimulante, l'hormone lutéinisante, le facteur de croissance transformant α, le facteur de croissance épidermique, le facteur de croissance analogue à l'insuline I et II, le facteur antagoniste de la matosematostatine, l'IGA, le glucose, le cholestérol, la caféine et la transcortine.

67. Appareil selon la revendication 41, dans lequel au moins un desdits réactifs est un marqueur détectable.

68. Appareil selon la revendication 67, dans lequel ledit marqueur détectable est choisi dans le groupe constitué par les billes magnétiques, les colorants fluorescents, les enzymes et les marqueurs colorimétriques.

69. Appareil selon la revendication 68, dans lequel lesdits colorants fluorescents sont choisis dans le groupe constitué par : l'isothiocyanate de fluorescéine, le rouge texas, la rhodamine, la protéine à fluorescence verte.

70. Appareil selon la revendication 68, dans lequel un desdits marqueurs colorimétriques est de l'or colloïdal.

71. Appareil selon la revendication 41, dans lequel lesdits analytes sont des anticorps choisis dans le groupe constitué de : les anticorps VIH, les anticorps HTLV, les anticorps *Helicobacter pylori,* les anticorps de l'hépatite, les anticorps des oreillons, les anticorps de la rubéole, la cotinine, la cocaïne, la benzoylecgonine, la benzodiazépine, le tétrahydrocannabinol, la nicotine, le théophylline éthanol, la phénytoïne, le paracétamol, le lithium, le diazépam, la nortrypyline, le sécobarbital, le phénobarbitol, la théophylline, la testostérone, l'oestradiol, 1a 17-hydroxyprogestérone, la progestérone, la thyroxine, la thyrolibérine, l'hormone folliculostimulante, l'hormone lutéinisante, le facteur de croissance transformant α, le facteur de croissance épidermique, le facteur de croissance analogue à l'insuline I et II, le facteur antagoniste de la matosematostatine, l'IGA, le glucose, le cholestérol, la caféine et la transcortine.

72. Dispositif de récolte et dosage de fluide, comprenant :
un logement ayant une extrémité de réception d'un fluide et une partie de dosage ;
une bande de dosage par immunochromatographie sur membrane contenue sensiblement dans la partie de dosage, la partie de dosage contenant au moins un réactif qui est utilisé pour détecter la présence et l'absence d'au moins un analyte dans un fluide ; et
une bande de récolte pour transporter le fluide d'une source de fluide vers la bande de dosage, la bande de récolte comprenant :
une première extrémité étroite contenue dans le logement et en communication fluidique avec la bande de dosage,
une seconde extrémité élargie faisant saillie de l'extrémité de réception du fluide ; et
une bande de blocage couplée entre et en communication fluidique avec la bande de dosage par immunochromatographie sur membrane et la bande de récolte.

73. Dispositif selon la revendication 72, dans lequel la bande de récolte comprend une matrice capillaire adaptée pour faire passer rapidement par capillarisation un fluide d'une source de fluide à la bande de dosage.

74. Dispositif selon la revendication 72, dans lequel la source de fluide est une cavité orale.

75. Dispositif selon la revendication 72, dans lequel la seconde extrémité est une d'une forme de pale et d'une forme sensiblement bulbeuse.

76. Dispositif pour le dosage d'un fluide oral, comprenant :
une partie de dosage logeant une bande de dosage par immunochromatographie sur membrane, la bande de dosage contenant au moins un réactif qui est utilisé pour détecter la présence et de l'absence d'au moins un analyte dans un fluide ; et
une partie col s'étendant depuis la partie de dosage, la partie col formant un canal pour la délivrance d'un fluide à la bande de dosage, le canal étant défini par une première partie étroite proximale à la partie de dosage et une seconde partie comprenant une ouverture pour la réception du fluide oral, dans laquelle la seconde partie comprend une largeur de canal qui est sensiblement plus large que la largeur de canal au niveau de l'extrémité étroite ;
une bande de récolte en communication fluidique avec la bande de dosage par immunochromatographie sur membrane, la bande de récolte ayant une première partie disposée dans le canal et une seconde partie faisant saillie vers l'extérieur depuis l'ouverture de la partie col ; et
une bande de blocage couplée entre et en communication fluide avec la bande de dosage par immunochromatographie sur membrane et la bande de récolte.

77. Dispositif selon la revendication 76, dans lequel la seconde partie de l'élément permettant la capillarité a une forme de pale.

78. Dispositif selon la revendication 76, dans lequel la largeur de la partie col forme ainsi depuis la largeur de l'ouverture vers la largeur de l'extrémité étroite.

79. Dispositif selon la revendication 72, dans lequel la bande de dosage est une bande d'immunochromatographie.

80. Dispositif selon la revendication 72, comprenant en outre un indicateur de l'adéquation d'un échantillon.

81. Dispositif selon la revendication 72, dans lequel le réactif est un partenaire de liaison immunospécifique qui porte un marqueur détectable.

82. Dispositif selon la revendication 76, dans lequel le réactif est un partenaire de liaison marqué par enzyme.

83. Dispositif selon la revendication 72, dans lequel le réactif comprend un antigène et un anticorps.
